# EUROPEAN PATENT APPLICATION

(11) **EP 4 023 666 A1**
(43) Date of publication of application: **06.07.2022**
(21) Application number: 20901536.1
(22) Date of filing: 07.01.2020
(51) Int. Cl.: C07K 14/55, C07K 19/00, C12N 15/26, C12N 15/62, A61K 38/20, A61K 38/17, A61P 37/04

(54) **INTERLEUKIN-2 DERIVATIVE**

(30) Priority: 17.12.2019 CN 201911302355
(71) Applicant: Leto Laboratories Co., Ltd, Changping District Huilongguan Town Beijing 100026 (CN)
(72) Inventor: ZHAO, Yao, Beijing 100026 (CN); PENG, Lujia, Beijing 100026 (CN); GUO, Jianyun, Beijing 100026 (CN); ZHU, Xiaoting, Beijing 100026 (CN); ZHANG, Jianjun, Beijing 100026 (CN); WEI, Tingting, Beijing 100026 (CN); LIU, Huijie, Beijing 100026 (CN); ZHENG, Qian, Beijing 100026 (CN); WANG, Jishu, Beijing 100026 (CN); ZHANG, Wei, Beijing 100026 (CN)
(74) Representative: Sun, Yiming
(86) International application number: PCT/CN2020/070748
(87) International publication number: WO 2021/120350

(57) **Abstract**

Disclosed is an IL-2 derivative. By introducing at least one cysteine residue based on wild-type IL-2, the binding plane of the IL-2 derivative and α receptor subunit is partially or completely blocked, while the affinity of the IL-2 derivative to the complex of β and γ receptor subunits is basically retained. -A complex is also disclosed, which comprises: an IL-2 derivative, which is introduced with a third cysteine residue based on wild-type IL-2; and a blocking module, which has or is introduced with a fourth cysteine residue; wherein the third cysteine residue on the IL-2 derivative and the fourth cysteine residue on the blocking module are capable of forming an intermolecular disulfide bond, thereby forming a complex of the IL-2 derivative and the blocking module; and wherein the binding plane of the IL-2 derivative and α receptor subunit is partially or completely blocked, while the affinity of the IL-2 derivative to the complex of β and γ receptor subunits is basically retained.

SEQUENCE LISTING

<110>  LETO LABORATORIES CO., LTD

<120>  INTERLEUKIN-2 DERIVATIVE

<160>  146

<170>  PatentIn version 3.5

<210>  1
<211>  133
<212>  PRT
<213>  Homo sapiens

<400>  1


<210>  2
<211>  133
<212>  PRT
<213>  Artificial Sequence

<400>  2

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210> 3
<211> 133
<212> PRT
<213> Artificial Sequence

<400> 3

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Cys Leu Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Cys Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  4
<211>  133
<212>  PRT
<213>  Artificial Sequence

<400>  4

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Phe Lys Cys Tyr Met Pro Lys
        35                  40                  45

Lys Ala Thr Glu Leu Lys His Leu Gln Cys Leu Glu Glu Cys Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  5
<211>  133
<212>  PRT
<213>  Artificial Sequence

<400>  5

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Phe Lys Cys Tyr Met Pro Lys
        35                  40                  45

Lys Ala Thr Glu Leu Lys His Leu Gln Cys Leu Glu Glu Glu Leu Lys
    50                  55                  60

Cys Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  6
<211>  133
<212>  PRT
<213>  Artificial Sequence

<400>  6

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Cys Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Cys Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  7
<211>  133
<212>  PRT
<213>  Artificial Sequence

<400>  7

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Phe Lys Phe Tyr Met Pro Lys
        35                  40                  45

Lys Ala Thr Glu Leu Lys His Leu Gln Cys Leu Glu Cys Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Cys Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  8
<211>  133
<212>  PRT
<213>  Artificial Sequence

<400>  8

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Cys Lys Phe Tyr Met Pro Lys
        35                  40                  45

Lys Ala Thr Glu Leu Lys His Leu Gln Cys Leu Glu Glu Glu Leu Lys
    50                  55                  60

Cys Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  9
<211>  133
<212>  PRT
<213>  Artificial Sequence

<400>  9

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Cys Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu

65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Cys Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  10
<211>  133
<212>  PRT
<213>  Artificial Sequence

<400>  10

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Cys Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Cys
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  11

<211>  133
<212>  PRT
<213>  Artificial Sequence

<400>  11

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Cys Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Cys Ile Val Glu Phe Leu Asn Arg Trp Ile Thr Phe Ala Gln Ser Ile
        115                 120                 125

Ile Ser Thr Leu Thr
    130


<210>  12
<211>  133
<212>  PRT
<213>  Artificial Sequence

<400>  12

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Cys Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Cys
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  13
<211>  133
<212>  PRT
<213>  Artificial Sequence

<400>  13

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Cys Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Cys
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  14
<211>  133
<212>  PRT
<213>  Artificial Sequence

<400>  14

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Cys Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Cys Ile Val Glu Phe Leu Asn Arg Trp Ile Thr Phe Ala Gln Ser Ile
        115                 120                 125

Ile Ser Thr Leu Thr
    130


<210>  15
<211>  133
<212>  PRT
<213>  Artificial Sequence

<400>  15

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Cys Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Cys Val Glu Phe Leu Asn Arg Trp Ile Thr Phe Ala Gln Ser Ile
        115                 120                 125

Ile Ser Thr Leu Thr
    130

<210>  16
<211>  133
<212>  PRT
<213>  Artificial Sequence

<400>  16

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Cys Leu Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Cys Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  17
<211>  133
<212>  PRT
<213>  Artificial Sequence

<400>  17

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Cys Leu Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Cys Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  18
<211>  133
<212>  PRT
<213>  Artificial Sequence

<400>  18

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Cys Met Leu Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Cys Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  19
<211>  133
<212>  PRT
<213>  Artificial Sequence

<400>  19

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Cys Met Leu Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Cys Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  20
<211>  133
<212>  PRT
<213>  Artificial Sequence

<400>  20

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Cys Thr Arg Met Leu Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Cys Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  21
<211>  133
<212>  PRT
<213>  Artificial Sequence

<400>  21

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys

20                  25                  30

Asn Pro Lys Cys Thr Arg Met Leu Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Cys Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  22
<211>  133
<212>  PRT
<213>  Artificial Sequence

<400>  22

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Cys Thr Arg Met Leu Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Cys Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala

            100                 105                 110

Thr Ile Val Glu Phe Leu Asn Arg Trp Ile Thr Phe Ala Gln Ser Ile
        115                 120                 125

Ile Ser Thr Leu Thr
    130

<210> 23
<211> 133
<212> PRT
<213> Artificial Sequence

<400> 23

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Cys Leu Thr Arg Met Leu Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Cys Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210> 24
<211> 133
<212> PRT
<213> Artificial Sequence

<400> 24

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Phe Cys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Cys
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  25
<211>  165
<212>  PRT
<213>  Artificial Sequence

<400>  25

Glu Leu Cys Asp Asp Asp Pro Pro Glu Ile Pro His Ala Thr Phe Lys
1               5                   10                  15

Ala Met Ala Tyr Lys Glu Gly Thr Met Leu Asn Cys Glu Cys Lys Arg
            20                  25                  30

Gly Phe Arg Arg Ile Lys Ser Gly Ser Leu Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Ile Tyr His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165

<210>  26
<211>  133
<212>  PRT
<213>  Artificial Sequence

<400>  26

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Cys Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  27
<211>  133
<212>  PRT
<213>  Artificial Sequence

<400>  27

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Cys Lys Leu Thr Arg Met Leu Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  28
<211>  133
<212>  PRT
<213>  Artificial Sequence

<400>  28

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Cys Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  29
<211>  133
<212>  PRT
<213>  Artificial Sequence

<400>  29

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Phe Lys Phe Cys Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  30
<211>  133
<212>  PRT
<213>  Artificial Sequence

<400>  30

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Cys Met Leu Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  31
<211>  133
<212>  PRT
<213>  Artificial Sequence

<400>  31

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Cys Met Leu Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Cys Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  32
<211>  133
<212>  PRT
<213>  Artificial Sequence

<400>  32

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Phe Lys Phe Tyr Met Pro Lys
        35                  40                  45

Lys Ala Thr Glu Leu Lys His Leu Gln Cys Leu Glu Cys Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  33
<211>  133
<212>  PRT
<213>  Artificial Sequence

<400>  33

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Cys Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  34
<211>  133
<212>  PRT
<213>  Artificial Sequence

<400>  34

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys

20                          25                          30

Asn Pro Cys Leu Thr Arg Met Leu Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  35
<211>  133
<212>  PRT
<213>  Artificial Sequence

<400>  35

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Cys Arg Met Leu Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala

100                 105                 110

Thr Ile Val Glu Phe Leu Asn Arg Trp Ile Thr Phe Ala Gln Ser Ile
        115                 120                 125

Ile Ser Thr Leu Thr
    130

<210>  36
<211>  133
<212>  PRT
<213>  Artificial Sequence

<400>  36

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Cys Met Leu Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  37
<211>  133
<212>  PRT
<213>  Artificial Sequence

<400>  37

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Cys Met Leu Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  38
<211>  133
<212>  PRT
<213>  Artificial Sequence

<400>  38

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Cys Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  39
<211>  133
<212>  PRT
<213>  Artificial Sequence

<400>  39

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Cys Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  40
<211>  133
<212>  PRT
<213>  Artificial Sequence

<400>  40

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Phe Cys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  41
<211>  133
<212>  PRT
<213>  Artificial Sequence

<400>  41

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Phe Cys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  42
<211>  133
<212>  PRT
<213>  Artificial Sequence

<400>  42

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Phe Lys Cys Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  43
<211>  133
<212>  PRT

<213>  Artificial Sequence

<400>  43

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Phe Cys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  44
<211>  133
<212>  PRT
<213>  Artificial Sequence

<400>  44

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Phe Lys Phe Tyr Met Pro Lys
        35                  40                  45

Lys Ala Thr Glu Leu Lys His Leu Gln Cys Leu Glu Cys Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  45
<211>  133
<212>  PRT
<213>  Artificial Sequence

<400>  45

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Phe Lys Phe Tyr Met Pro Lys
        35                  40                  45

Lys Ala Thr Glu Leu Lys His Leu Gln Cys Leu Glu Glu Cys Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  46
<211>  133
<212>  PRT
<213>  Artificial Sequence

<400>  46

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Phe Lys Phe Tyr Met Pro Lys
        35                  40                  45

Lys Ala Thr Glu Leu Lys His Leu Gln Cys Leu Glu Glu Glu Leu Cys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130


<210>  47
<211>  133
<212>  PRT
<213>  Artificial Sequence

<400>  47

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Phe Lys Phe Tyr Met Pro Lys
        35                  40                  45

Lys Ala Thr Glu Leu Lys His Leu Gln Cys Leu Glu Glu Glu Leu Cys

50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  48
<211>  133
<212>  PRT
<213>  Artificial Sequence

<400>  48

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Phe Lys Phe Tyr Met Pro Lys
        35                  40                  45

Lys Ala Thr Glu Leu Lys His Leu Gln Cys Leu Glu Glu Glu Leu Cys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr

    130

<210>  49
<211>  133
<212>  PRT
<213>  Artificial Sequence

<400>  49

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Cys Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ala Gln Ser Ile
        115                 120                 125

Ile Ser Thr Leu Thr
    130

<210>  50
<211>  165
<212>  PRT
<213>  Artificial Sequence

<400>  50

Glu Leu Cys Asp Asp Asp Pro Pro Glu Ile Pro His Ala Thr Phe Lys
1               5                   10                  15

Ala Met Ala Tyr Lys Glu Gly Thr Met Leu Cys Cys Glu Cys Lys Arg
            20                  25                  30

Gly Phe Arg Arg Ile Lys Ser Gly Ser Leu Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Ile Tyr His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165

<210>  51
<211>  165
<212>  PRT
<213>  Artificial Sequence

<400>  51

Glu Leu Cys Cys Asp Asp Pro Pro Glu Ile Pro His Ala Thr Phe Lys
1               5                   10                  15

Ala Met Ala Tyr Lys Glu Gly Thr Met Leu Asn Cys Glu Cys Lys Arg
            20                  25                  30

Gly Phe Arg Arg Ile Lys Ser Gly Ser Leu Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Ile Tyr His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165

<210> 52
<211> 165
<212> PRT
<213> Artificial Sequence

<400> 52

Glu Leu Cys Asp Asp Asp Pro Pro Glu Ile Pro His Ala Thr Phe Lys
1               5                   10                  15

Ala Met Ala Tyr Lys Glu Gly Thr Met Leu Asn Cys Glu Cys Lys Arg
            20                  25                  30

Gly Phe Arg Arg Ile Lys Ser Gly Ser Cys Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Ile Tyr His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165

<210>  53
<211>  165
<212>  PRT
<213>  Artificial Sequence

<400>  53

Glu Leu Cys Asp Asp Asp Pro Pro Glu Ile Pro His Ala Thr Phe Lys
1               5                   10                  15

Ala Met Ala Tyr Lys Glu Gly Thr Met Leu Asn Cys Glu Cys Lys Arg
            20                  25                  30

Gly Phe Cys Arg Ile Lys Ser Gly Ser Leu Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Ile Tyr His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165

<210>  54
<211>  165

<212>  PRT
<213>  Artificial Sequence

<400>  54

Glu Leu Cys Asp Asp Asp Pro Pro Glu Ile Pro His Ala Thr Phe Lys
1               5                   10                  15

Ala Met Ala Tyr Lys Glu Gly Thr Met Leu Asn Cys Glu Cys Lys Arg
            20                  25                  30

Gly Phe Arg Arg Ile Lys Ser Gly Ser Leu Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Ile Tyr Cys Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165


<210>  55
<211>  165
<212>  PRT
<213>  Artificial Sequence

<400>  55

Glu Leu Cys Asp Asp Asp Pro Pro Glu Ile Pro His Ala Thr Phe Lys
1               5                   10                  15

Ala Met Ala Tyr Lys Glu Gly Thr Cys Leu Asn Cys Glu Cys Lys Arg
            20                  25                  30

Gly Phe Arg Arg Ile Lys Ser Gly Ser Leu Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Ile Tyr His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165

<210>  56
<211>  165
<212>  PRT
<213>  Artificial Sequence

<400>  56

Glu Leu Cys Asp Asp Asp Pro Pro Glu Ile Pro His Ala Thr Phe Lys
1               5                   10                  15

Ala Met Ala Tyr Lys Glu Gly Thr Met Leu Asn Cys Glu Cys Lys Arg
            20                  25                  30

Gly Phe Arg Arg Ile Lys Cys Gly Ser Leu Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Ile Tyr His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165

<210>  57
<211>  165
<212>  PRT
<213>  Artificial Sequence

<400>  57

Glu Leu Cys Asp Asp Asp Pro Pro Glu Ile Pro His Ala Thr Phe Lys
1               5                   10                  15

Ala Met Ala Tyr Lys Glu Gly Thr Met Leu Asn Cys Glu Cys Lys Arg
            20                  25                  30

Gly Phe Arg Arg Ile Lys Ser Gly Ser Leu Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Cys Tyr His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165

<210>  58
<211>  165
<212>  PRT
<213>  Artificial Sequence

<400>  58

Glu Leu Cys Cys Asp Asp Pro Pro Glu Ile Pro His Ala Thr Phe Lys
1               5                   10                  15

Ala Met Ala Tyr Lys Glu Gly Thr Met Leu Asn Cys Glu Cys Lys Arg
            20                  25                  30

Gly Phe Arg Arg Ile Lys Ser Gly Ser Leu Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Ile Tyr His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165

<210>  59
<211>  165
<212>  PRT
<213>  Artificial Sequence

<400>  59

Glu Leu Cys Cys Asp Asp Pro Pro Glu Ile Pro His Ala Thr Phe Lys
1               5                   10                  15

Ala Met Ala Tyr Lys Glu Gly Thr Met Leu Asn Cys Glu Cys Lys Arg
            20                  25                  30

Gly Phe Arg Arg Ile Lys Ser Gly Ser Leu Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Ile Tyr His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165


<210>  60
<211>  165
<212>  PRT
<213>  Artificial Sequence

<400>  60

Glu Leu Cys Cys Asp Asp Pro Pro Glu Ile Pro His Ala Thr Phe Lys
1               5                   10                  15

Ala Met Ala Tyr Lys Glu Gly Thr Met Leu Asn Cys Glu Cys Lys Arg
            20                  25                  30

Gly Phe Arg Arg Ile Lys Ser Gly Ser Leu Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Ile Tyr His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165

<210> 61
<211> 165
<212> PRT
<213> Artificial Sequence

<400> 61

Glu Leu Cys Asp Cys Asp Pro Pro Glu Ile Pro His Ala Thr Phe Lys
1               5                   10                  15

Ala Met Ala Tyr Lys Glu Gly Thr Met Leu Asn Cys Glu Cys Lys Arg
            20                  25                  30

Gly Phe Arg Arg Ile Lys Ser Gly Ser Leu Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Ile Tyr His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165

<210>  62
<211>  165
<212>  PRT
<213>  Artificial Sequence

<400>  62

Glu Leu Cys Asp Asp Asp Pro Pro Glu Ile Pro His Ala Thr Phe Lys
1               5                   10                  15

Ala Met Ala Tyr Lys Glu Gly Thr Met Leu Asn Cys Glu Cys Lys Arg
            20                  25                  30

Gly Phe Arg Arg Ile Lys Ser Gly Ser Cys Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Ile Tyr His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165

<210>  63
<211>  165
<212>  PRT
<213>  Artificial Sequence

<400>  63

Glu Leu Cys Asp Asp Asp Pro Pro Glu Ile Pro His Ala Thr Phe Lys
1               5                   10                  15

Ala Met Ala Tyr Lys Glu Gly Thr Met Leu Asn Cys Glu Cys Lys Arg
            20                  25                  30

Gly Phe Arg Arg Ile Lys Ser Gly Ser Leu Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Ile Cys His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165

<210>  64
<211>  165
<212>  PRT
<213>  Artificial Sequence

<400>  64

Glu Leu Cys Asp Asp Asp Pro Pro Glu Ile Pro His Ala Thr Phe Lys
1               5                   10                  15

Ala Met Ala Tyr Lys Glu Gly Thr Met Leu Asn Cys Glu Cys Lys Arg
            20                  25                  30

Gly Phe Cys Arg Ile Lys Ser Gly Ser Leu Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Ile Tyr His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165

<210>  65
<211>  165
<212>  PRT
<213>  Artificial Sequence

<400>  65

Glu Leu Cys Asp Asp Asp Pro Pro Glu Ile Pro His Ala Thr Phe Lys

1               5                   10                  15

Ala Met Ala Tyr Lys Glu Gly Thr Met Leu Asn Cys Glu Cys Lys Arg
            20                  25                  30

Gly Phe Arg Cys Ile Lys Ser Gly Ser Leu Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Ile Tyr His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165

<210>  66
<211>  165
<212>  PRT
<213>  Artificial Sequence

<400>  66

Glu Leu Cys Asp Asp Asp Pro Pro Glu Ile Pro His Ala Thr Phe Lys
1               5                   10                  15

Ala Met Ala Tyr Lys Glu Gly Thr Met Leu Asn Cys Glu Cys Lys Arg
            20                  25                  30

Gly Phe Arg Arg Ile Lys Ser Gly Ser Cys Tyr Met Leu Cys Thr Gly
        35                  40                  45

Asn Ser Ser His Ser Ser Trp Asp Asn Gln Cys Gln Cys Thr Ser Ser

50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Ile Tyr His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165

<210>  67
<211>  165
<212>  PRT
<213>  Artificial Sequence

<400>  67

Glu Leu Cys Asp Asp Asp Pro Pro Glu Ile Pro His Ala Thr Phe Lys
1               5                   10                  15

Ala Met Ala Tyr Lys Glu Gly Thr Met Leu Asn Cys Glu Cys Lys Arg
            20                  25                  30

Gly Phe Arg Arg Ile Lys Ser Gly Ser Cys Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn

            100                 105                 110

Glu Ala Thr Glu Arg Ile Tyr His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165

<210> 68
<211> 165
<212> PRT
<213> Artificial Sequence

<400> 68

Glu Leu Cys Asp Asp Asp Pro Pro Glu Ile Pro His Ala Thr Phe Lys
1               5                   10                  15

Ala Met Ala Tyr Lys Glu Gly Thr Met Leu Asn Cys Glu Cys Lys Arg
            20                  25                  30

Gly Phe Arg Arg Ile Cys Ser Gly Ser Leu Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Ile Tyr His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln

145                 150                 155                 160

Leu Ile Cys Thr Gly
                165

<210>  69
<211>  165
<212>  PRT
<213>  Artificial Sequence

<400>  69

Glu Leu Cys Asp Asp Asp Pro Pro Glu Ile Pro His Ala Thr Phe Lys
1               5                   10                  15

Ala Met Ala Tyr Lys Glu Gly Thr Met Leu Asn Cys Glu Cys Lys Arg
            20                  25                  30

Gly Phe Arg Arg Ile Cys Ser Gly Ser Leu Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Ile Tyr His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165

<210>  70
<211>  165
<212>  PRT
<213>  Artificial Sequence

<400>  70

Glu Leu Cys Asp Asp Asp Pro Pro Glu Ile Pro His Ala Thr Phe Lys
1               5                   10                  15

Ala Met Ala Tyr Lys Glu Gly Thr Met Leu Asn Cys Glu Cys Lys Arg
            20                  25                  30

Gly Phe Arg Arg Ile Lys Cys Gly Ser Leu Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Ile Tyr His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165


<210>  71
<211>  165
<212>  PRT
<213>  Artificial Sequence

<400>  71

Glu Leu Cys Asp Asp Asp Pro Pro Glu Ile Pro His Ala Thr Phe Lys
1               5                   10                  15

Ala Met Ala Tyr Lys Glu Gly Thr Met Leu Asn Cys Glu Cys Lys Arg
            20                  25                  30

Gly Phe Arg Arg Ile Lys Ser Cys Ser Leu Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Ile Tyr His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165

<210>  72
<211>  165
<212>  PRT
<213>  Artificial Sequence

<400>  72

Glu Leu Cys Asp Asp Asp Pro Pro Glu Ile Pro His Ala Thr Phe Lys
1               5                   10                  15

Ala Met Ala Tyr Lys Glu Gly Thr Met Leu Asn Cys Glu Cys Lys Arg
            20                  25                  30

Gly Phe Arg Arg Ile Lys Ser Gly Cys Leu Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Ile Tyr His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165

<210> 73
<211> 165
<212> PRT
<213> Artificial Sequence

<400> 73

Glu Leu Cys Asp Asp Asp Pro Pro Glu Ile Pro His Ala Thr Phe Lys
1               5                   10                  15

Ala Met Ala Tyr Lys Glu Gly Thr Met Leu Asn Cys Glu Cys Lys Arg
            20                  25                  30

Gly Phe Arg Arg Ile Cys Ser Gly Ser Leu Tyr Met Leu Cys Thr Gly
        35                  40                  45

Asn Ser Ser His Ser Ser Trp Asp Asn Gln Cys Gln Cys Thr Ser Ser
    50                  55                  60

Ala Thr Arg Asn Thr Thr Lys Gln Val Thr Pro Gln Pro Glu Glu Gln
65                  70                  75                  80

Lys Glu Arg Lys Thr Thr Glu Met Gln Ser Pro Met Gln Pro Val Asp
                85                  90                  95

Gln Ala Ser Leu Pro Gly His Cys Arg Glu Pro Pro Pro Trp Glu Asn
            100                 105                 110

Glu Ala Thr Glu Arg Ile Tyr His Phe Val Val Gly Gln Met Val Tyr
        115                 120                 125

Tyr Gln Cys Val Gln Gly Tyr Arg Ala Leu His Arg Gly Pro Ala Glu
    130                 135                 140

Ser Val Cys Lys Met Thr His Gly Lys Thr Arg Trp Thr Gln Pro Gln
145                 150                 155                 160

Leu Ile Cys Thr Gly
                165

<210>  74
<211>  399
<212>  DNA
<213>  Artificial Sequence

<400>  74
gcccccacaa gctccagcac caagaagaca cagctgcagc tggaacatct gctgctggac      60

ctccagatga ttctgaacgg catcaacaac tacaagaatc ccaagctgac aagaatgctc     120

accttcaagt tctacatgcc caagaaggcc accgagctga agcatctgca gtgtctggag     180

gaggagctga agcctctgga agaggtgctg aatctggccc agtccaagaa cttccatctg     240

aggcctagag atctgatcag caacatcaac gtcatcgtgc tggagctgaa gggcagcgag     300

accaccttca tgtgcgagta cgccgatgag accgccacca tcgtggagtt tctgaataga     360

tggatcacct tcgcccagag catcatcagc acactgacc                            399

<210>  75
<211>  399
<212>  DNA
<213>  Artificial Sequence

<400>  75
gcccccacaa gctccagcac caagaagaca cagctgcagc tggaacatct gctgctggac      60

ctccagatga ttctgaacgg catcaacaac tacaagaatc ccaagctgac aagatgcctc     120

accttcaagt tctacatgcc caagaaggcc accgagctga agcatctgca gtgtctggag     180

gaggagctga agcctctgga agagtgtctg aatctggccc agtccaagaa cttccatctg     240

aggcctagag atctgatcag caacatcaac gtcatcgtgc tggagctgaa gggcagcgag     300

accaccttca tgtgcgagta cgccgatgag accgccacca tcgtggagtt tctgaataga     360

tggatcacct tcgcccagag catcatcagc acactgacc                            399

<210>  76
<211>  399
<212>  DNA
<213>  Artificial Sequence

<400>  76
gcccccacaa gctccagcac caagaagaca cagctgcagc tggaacatct gctgctggac      60

ctccagatga ttctgaacgg catcaacaac tacaagaatc ccaagctgac aagaatgctc     120

accttcaagt gctacatgcc caagaaggcc accgagctga agcatctgca gtgtctggag     180

gagtgtctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 77
<211> 399
<212> DNA
<213> Artificial Sequence

<400> 77
gcccccacaa gctccagcac caagaagaca cagctgcagc tggaacatct gctgctggac 60

ctccagatga ttctgaacgg catcaacaac tacaagaatc ccaagctgac aagaatgctc 120

accttcaagt gctacatgcc caagaaggcc accgagctga agcatctgca gtgtctggag 180

gaggagctga agtgtctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 78
<211> 399
<212> DNA
<213> Artificial Sequence

<400> 78
gcccccacaa gctccagcac caagaagaca cagctgcagc tggaacatct gctgctggac 60

ctccagatga ttctgaacgg catcaacaac tacaagaatc ccaagctgac aagaatgctc 120

acctgcaagt tctacatgcc caagaaggcc accgagctga agcatctgca gtgtctggag 180

gaggagctga agcctctgga agagtgtctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 79
<211> 399
<212> DNA
<213> Artificial Sequence

<400> 79
gcccccacaa gctccagcac caagaagaca cagctgcagc tggaacatct gctgctggac 60

ctccagatga ttctgaacgg catcaacaac tacaagaatc ccaagctgac aagaatgctc 120

accttcaagt tctacatgcc caagaaggcc accgagctga agcatctgca gtgtctggag 180

tgcgagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagtg tgccgatgag accgccacca tcgtggagtt tctgaataga 360

tggatcacct tcgcccagag catcatcagc acactgacc 399

<210> 80
<211> 399
<212> DNA
<213> Artificial Sequence

<400> 80
gcccccacaa gctccagcac caagaagaca cagctgcagc tggaacatct gctgctggac 60

ctccagatga ttctgaacgg catcaacaac tacaagaatc ccaagctgac aagaatgctc 120

acctgcaagt tctacatgcc caagaaggcc accgagctga agcatctgca gtgtctggag 180

gaggagctga agtgtctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 81
<211> 399
<212> DNA
<213> Artificial Sequence

<400> 81
gcccccacaa gctccagcac caagaagaca cagctgcagc tggaacatct gctgctggac 60

ctccagatga ttctgaacgg catcaacaac tacaagaatc ccaagctgac aagaatgctc 120

acctgcaagt tctacatgcc caagaaggcc accgagctga agcatctgca gtgtctggag 180

gaggagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag tgtgccacca tcgtggagtt tctgaataga 360

tggatcacct tcgcccagag catcatcagc acactgacc 399

<210> 82
<211> 399
<212> DNA
<213> Artificial Sequence

<400> 82
gcccccacaa gctccagcac caagaagaca cagctgcagc tggaacatct gctgctggac 60

ctccagatga ttctgaacgg catcaacaac tacaagaatc ccaagctgac aagaatgctc 120

acctgcaagt tctacatgcc caagaaggcc accgagctga agcatctgca gtgtctggag 180

gaggagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag acctgtacca tcgtggagtt tctgaataga 360

tggatcacct tcgcccagag catcatcagc acactgacc 399

<210> 83
<211> 399
<212> DNA
<213> Artificial Sequence

<400> 83
gcccccacaa gctccagcac caagaagaca cagctgcagc tggaacatct gctgctggac 60

ctccagatga ttctgaacgg catcaacaac tacaagaatc ccaagctgac aagaatgctc 120

acctgcaagt tctacatgcc caagaaggcc accgagctga agcatctgca gtgtctggag 180

gaggagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgcctgta tcgtggagtt tctgaataga 360

tggatcacct tcgcccagag catcatcagc acactgacc 399

<210> 84
<211> 399
<212> DNA
<213> Artificial Sequence

<400> 84
gcccccacaa gctccagcac caagaagaca cagctgcagc tggaacatct gctgctggac 60

ctccagatga ttctgaacgg catcaacaac tacaagaatc ccaagctgac aagaatgctc 120

tgcttcaagt tctacatgcc caagaaggcc accgagctga agcatctgca gtgtctggag 180

gaggagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag acctgtacca tcgtggagtt tctgaataga 360

tggatcacct tcgcccagag catcatcagc acactgacc 399

<210> 85
<211> 399
<212> DNA
<213> Artificial Sequence

<400> 85
gcccccacaa gctccagcac caagaagaca cagctgcagc tggaacatct gctgctggac 60

ctccagatga ttctgaacgg catcaacaac tacaagaatc ccaagctgac aagaatgtgc 120

accttcaagt tctacatgcc caagaaggcc accgagctga agcatctgca gtgtctggag 180

gaggagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag acctgtacca tcgtggagtt tctgaataga 360

tggatcacct tcgcccagag catcatcagc acactgacc 399

<210> 86
<211> 399
<212> DNA
<213> Artificial Sequence

<400> 86
gcccccacaa gctccagcac caagaagaca cagctgcagc tggaacatct gctgctggac 60

ctccagatga ttctgaacgg catcaacaac tacaagaatc ccaagctgac aagaatgtgc 120

accttcaagt tctacatgcc caagaaggcc accgagctga agcatctgca gtgtctggag 180

gaggagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgcctgta tcgtggagtt tctgaataga 360

tggatcacct tcgcccagag catcatcagc acactgacc 399

<210> 87
<211> 399
<212> DNA
<213> Artificial Sequence

<400> 87
gcccccacaa gctccagcac caagaagaca cagctgcagc tggaacatct gctgctggac 60

ctccagatga ttctgaacgg catcaacaac tacaagaatc ccaagctgac aagaatgtgc 120

accttcaagt tctacatgcc caagaaggcc accgagctga agcatctgca gtgtctggag 180

gaggagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacct gtgtggagtt tctgaataga 360

tggatcacct tcgcccagag catcatcagc acactgacc 399

<210> 88
<211> 399
<212> DNA
<213> Artificial Sequence

<400> 88
gcccccacaa gctccagcac caagaagaca cagctgcagc tggaacatct gctgctggac 60

ctccagatga ttctgaacgg catcaacaac tacaagaatc ccaagctgac aagatgcctc 120

accttcaagt tctacatgcc caagaaggcc accgagctga agcatctgca gtgtctggag 180

gaggagctga agcctctgga agaggtgctg aattgtgccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 89
<211> 399
<212> DNA
<213> Artificial Sequence

<400> 89
gcccccacaa gctccagcac caagaagaca cagctgcagc tggaacatct gctgctggac 60

ctccagatga ttctgaacgg catcaacaac tacaagaatc ccaagctgac aagatgcctc 120

accttcaagt tctacatgcc caagaaggcc accgagctga agcatctgca gtgtctggag 180

gaggagctga agcctctgga agaggtgctg aatctgtgtc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 90
<211> 399
<212> DNA
<213> Artificial Sequence

<400> 90
gcccccacaa gctccagcac caagaagaca cagctgcagc tggaacatct gctgctggac 60

ctccagatga ttctgaacgg catcaacaac tacaagaatc ccaagctgac atgcatgctc 120

accttcaagt tctacatgcc caagaaggcc accgagctga agcatctgca gtgtctggag 180

gaggagctga agcctctgga agagtgtctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 91
<211> 399
<212> DNA
<213> Artificial Sequence

<400> 91
gcccccacaa gctccagcac caagaagaca cagctgcagc tggaacatct gctgctggac 60

ctccagatga ttctgaacgg catcaacaac tacaagaatc ccaagctgac atgcatgctc 120

accttcaagt tctacatgcc caagaaggcc accgagctga agcatctgca gtgtctggag 180

gaggagctga agcctctgga agaggtgctg aattgtgccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 92
<211> 399
<212> DNA
<213> Artificial Sequence

<400> 92
gcccccacaa gctccagcac caagaagaca cagctgcagc tggaacatct gctgctggac 60

ctccagatga ttctgaacgg catcaacaac tacaagaatc ccaagtgcac aagaatgctc 120

accttcaagt tctacatgcc caagaaggcc accgagctga agcatctgca gtgtctggag 180

gaggagctga agcctctgga agagtgtctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 93
<211> 399
<212> DNA
<213> Artificial Sequence

<400> 93
gcccccacaa gctccagcac caagaagaca cagctgcagc tggaacatct gctgctggac 60

ctccagatga ttctgaacgg catcaacaac tacaagaatc ccaagtgcac aagaatgctc 120

accttcaagt tctacatgcc caagaaggcc accgagctga agcatctgca gtgtctggag 180

gaggagctga agcctctgga agaggtgctg aattgtgccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 94
<211> 399
<212> DNA
<213> Artificial Sequence

<400> 94
gcccccacaa gctccagcac caagaagaca cagctgcagc tggaacatct gctgctggac 60

ctccagatga ttctgaacgg catcaacaac tacaagaatc ccaagtgcac aagaatgctc 120

accttcaagt tctacatgcc caagaaggcc accgagctga agcatctgca gtgtctggag 180

gaggagctga agcctctgga agaggtgctg aatctgtgtc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 95
<211> 399
<212> DNA
<213> Artificial Sequence

<400> 95
gcccccacaa gctccagcac caagaagaca cagctgcagc tggaacatct gctgctggac 60

ctccagatga ttctgaacgg catcaacaac tacaagaatc cctgcctgac aagaatgctc 120

accttcaagt tctacatgcc caagaaggcc accgagctga agcatctgca gtgtctggag 180

gaggagctga agcctctgga agagtgtctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 96
<211> 399
<212> DNA
<213> Artificial Sequence

<400> 96
gcccccacaa gctccagcac caagaagaca cagctgcagc tggaacatct gctgctggac 60

ctccagatga ttctgaacgg catcaacaac tacaagaatc ccaagctgac aagaatgctc 120

accttctgct tctacatgcc caagaaggcc accgagctga agcatctgca gtgtctggag 180

gaggagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag acctgtacca tcgtggagtt tctgaataga 360

tggatcacct tcgcccagag catcatcagc acactgacc 399

<210> 97
<211> 399
<212> DNA
<213> Artificial Sequence

<400> 97
gcccccacaa gctccagcac caagaagaca cagctgcagc tggaacatct gctgctggac 60

ctccagatga ttctgaacgg catcaacaac tacaagaatc ccaagctgac aagaatgctc 120

tgcttcaagt tctacatgcc caagaaggcc accgagctga agcatctgca gtgtctggag 180

gaggagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 98
<211> 399
<212> DNA
<213> Artificial Sequence

<400> 98
gcccccacaa gctccagcac caagaagaca cagctgcagc tggaacatct gctgctggac 60

ctccagatga ttctgaacgg catcaacaac tacaagaatt gcaagctgac aagaatgctc 120

accttcaagt tctacatgcc caagaaggcc accgagctga agcatctgca gtgtctggag 180

gaggagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 99
<211> 399
<212> DNA
<213> Artificial Sequence

<400> 99
gcccccacaa gctccagcac caagaagaca cagctgcagc tggaacatct gctgctggac 60

ctccagatga ttctgaacgg catcaacaac tacaagaatc ccaagctgac aagaatgctc 120

accttcaagt tctacatgcc caagaaggcc accgagctga agcatctgca gtgtctggag 180

gaggagctga agcctctgga atgcgtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 100
<211> 399
<212> DNA
<213> Artificial Sequence

<400> 100
gcccccacaa gctccagcac caagaagaca cagctgcagc tggaacatct gctgctggac 60

ctccagatga ttctgaacgg catcaacaac tacaagaatc ccaagctgac aagaatgctc 120

accttcaagt tctgcatgcc caagaaggcc accgagctga agcatctgca gtgtctggag 180

gaggagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 101
<211> 399
<212> DNA
<213> Artificial Sequence

<400> 101
gcccccacaa gctccagcac caagaagaca cagctgcagc tggaacatct gctgctggac 60

ctccagatga ttctgaacgg catcaacaac tacaagaatc ccaagctgac atgcatgctc 120

accttcaagt tctacatgcc caagaaggcc accgagctga agcatctgca gtgtctggag 180

gaggagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 102
<211> 399
<212> DNA
<213> Artificial Sequence

<400> 102
gcccccacaa gctccagcac caagaagaca cagctgcagc tggaacatct gctgctggac 60

ctccagatga ttctgaacgg catcaacaac tacaagaatc ccaagctgac aagaatgctc 120

accttcaagt tctacatgcc caagaaggcc accgagctga agcatctgca gtgtctggag 180

gaggagctga agcctctgga agaggtgctg aattgcgccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 103
<211> 399
<212> DNA
<213> Artificial Sequence

<400> 103
gcccccacaa gctccagcac caagaagaca cagctgcagc tggaacatct gctgctggac 60

ctccagatga ttctgaacgg catcaacaac tacaagaatc ccaagctgac aagaatgctc 120

accttcaagt tctacatgcc caagaaggcc accgagctga agcatctgca gtgtctggag 180

tgcgagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 104
<211> 399
<212> DNA
<213> Artificial Sequence

<400> 104
gcccccacaa gctccagcac caagaagaca cagctgcagc tggaacatct gctgctggac 60

ctccagatga ttctgaacgg catcaacaac tacaagaatc ccaagctgac aagaatgctc 120

tgcttcaagt tctacatgcc caagaaggcc accgagctga agcatctgca gtgtctggag 180

gaggagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 105
<211> 399
<212> DNA
<213> Artificial Sequence

<400> 105
gcccccacaa gctccagcac caagaagaca cagctgcagc tggaacatct gctgctggac 60

ctccagatga ttctgaacgg catcaacaac tacaagaatc cctgcctgac aagaatgctc 120

accttcaagt tctacatgcc caagaaggcc accgagctga agcatctgca gtgtctggag 180

gaggagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 106
<211> 399
<212> DNA
<213> Artificial Sequence

<400> 106
gcccccacaa gctccagcac caagaagaca cagctgcagc tggaacatct gctgctggac 60

ctccagatga ttctgaacgg catcaacaac tacaagaatc ccaagctgtg cagaatgctc 120

accttcaagt tctacatgcc caagaaggcc accgagctga agcatctgca gtgtctggag 180

gaggagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 107
<211> 399
<212> DNA
<213> Artificial Sequence

<400> 107
gcccccacaa gctccagcac caagaagaca cagctgcagc tggaacatct gctgctggac 60

ctccagatga ttctgaacgg catcaacaac tacaagaatc ccaagctgac atgcatgctc 120

accttcaagt tctacatgcc caagaaggcc accgagctga agcatctgca gtgtctggag 180

gaggagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 108
<211> 399
<212> DNA
<213> Artificial Sequence

<400> 108
gcccccacaa gctccagcac caagaagaca cagctgcagc tggaacatct gctgctggac 60

ctccagatga ttctgaacgg catcaacaac tacaagaatc ccaagctgac atgcatgctc 120

accttcaagt tctacatgcc caagaaggcc accgagctga agcatctgca gtgtctggag 180

gaggagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 109
<211> 399
<212> DNA
<213> Artificial Sequence

<400> 109
gcccccacaa gctccagcac caagaagaca cagctgcagc tggaacatct gctgctggac 60

ctccagatga ttctgaacgg catcaacaac tacaagaatc ccaagctgac aagaatgctc 120

tgcttcaagt tctacatgcc caagaaggcc accgagctga agcatctgca gtgtctggag 180

gaggagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 110
<211> 399
<212> DNA
<213> Artificial Sequence

<400> 110
gcccccacaa gctccagcac caagaagaca cagctgcagc tggaacatct gctgctggac 60

ctccagatga ttctgaacgg catcaacaac tacaagaatc ccaagctgac aagaatgctc 120

tgcttcaagt tctacatgcc caagaaggcc accgagctga agcatctgca gtgtctggag 180

gaggagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 111
<211> 399
<212> DNA
<213> Artificial Sequence

<400> 111
gcccccacaa gctccagcac caagaagaca cagctgcagc tggaacatct gctgctggac 60

ctccagatga ttctgaacgg catcaacaac tacaagaatc ccaagctgac aagaatgctc 120

accttctgct tctacatgcc caagaaggcc accgagctga agcatctgca gtgtctggag 180

gaggagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 112
<211> 399
<212> DNA
<213> Artificial Sequence

<400> 112
gcccccacaa gctccagcac caagaagaca cagctgcagc tggaacatct gctgctggac 60

ctccagatga ttctgaacgg catcaacaac tacaagaatc ccaagctgac aagaatgctc 120

accttctgct tctacatgcc caagaaggcc accgagctga agcatctgca gtgtctggag 180

gaggagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 113
<211> 399
<212> DNA
<213> Artificial Sequence

<400> 113
gcccccacaa gctccagcac caagaagaca cagctgcagc tggaacatct gctgctggac 60

ctccagatga ttctgaacgg catcaacaac tacaagaatc ccaagctgac aagaatgctc 120

accttcaagt gctacatgcc caagaaggcc accgagctga agcatctgca gtgtctggag 180

gaggagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 114
<211> 399
<212> DNA
<213> Artificial Sequence

<400> 114
gcccccacaa gctccagcac caagaagaca cagctgcagc tggaacatct gctgctggac 60

ctccagatga ttctgaacgg catcaacaac tacaagaatc ccaagctgac aagaatgctc 120

accttctgct tctacatgcc caagaaggcc accgagctga agcatctgca gtgtctggag 180

gaggagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 115
<211> 399
<212> DNA
<213> Artificial Sequence

<400> 115
gcccccacaa gctccagcac caagaagaca cagctgcagc tggaacatct gctgctggac 60

ctccagatga ttctgaacgg catcaacaac tacaagaatc ccaagctgac aagaatgctc 120

accttcaagt tctacatgcc caagaaggcc accgagctga agcatctgca gtgtctggag 180

tgcgagctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 116
<211> 399
<212> DNA
<213> Artificial Sequence

<400> 116
gcccccacaa gctccagcac caagaagaca cagctgcagc tggaacatct gctgctggac 60

ctccagatga ttctgaacgg catcaacaac tacaagaatc ccaagctgac aagaatgctc 120

accttcaagt tctacatgcc caagaaggcc accgagctga agcatctgca gtgtctggag 180

gagtgcctga agcctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 117
<211> 399
<212> DNA
<213> Artificial Sequence

<400> 117
gcccccacaa gctccagcac caagaagaca cagctgcagc tggaacatct gctgctggac 60

ctccagatga ttctgaacgg catcaacaac tacaagaatc ccaagctgac aagaatgctc 120

accttcaagt tctacatgcc caagaaggcc accgagctga agcatctgca gtgtctggag 180

gaggagctgt gccctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 118
<211> 399
<212> DNA
<213> Artificial Sequence

<400> 118
gcccccacaa gctccagcac caagaagaca cagctgcagc tggaacatct gctgctggac 60

ctccagatga ttctgaacgg catcaacaac tacaagaatc ccaagctgac aagaatgctc 120

accttcaagt tctacatgcc caagaaggcc accgagctga agcatctgca gtgtctggag 180

gaggagctgt gccctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 119
<211> 399
<212> DNA
<213> Artificial Sequence

<400> 119
gcccccacaa gctccagcac caagaagaca cagctgcagc tggaacatct gctgctggac 60

ctccagatga ttctgaacgg catcaacaac tacaagaatc ccaagctgac aagaatgctc 120

accttcaagt tctacatgcc caagaaggcc accgagctga agcatctgca gtgtctggag 180

gaggagctgt gccctctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 120
<211> 399
<212> DNA
<213> Artificial Sequence

<400> 120
gcccccacaa gctccagcac caagaagaca cagctgcagc tggaacatct gctgctggac 60

ctccagatga ttctgaacgg catcaacaac tacaagaatc ccaagctgac aagaatgctc 120

accttcaagt tctacatgcc caagaaggcc accgagctga agcatctgca gtgtctggag 180

gaggagctga agtgcctgga agaggtgctg aatctggccc agtccaagaa cttccatctg 240

aggcctagag atctgatcag caacatcaac gtcatcgtgc tggagctgaa gggcagcgag 300

accaccttca tgtgcgagta cgccgatgag accgccacca tcgtggagtt tctgaataga 360

tggatcacct tcgcccagag catcatcagc acactgacc 399

<210> 121
<211> 495
<212> DNA
<213> Artificial Sequence

<400> 121
gagctctgcg acgatgatcc ccccgaaatc ccccacgcca cattcaaggc catggcctac 60

aaggagggaa ccatgctctg ttgcgagtgc aagaggggat ttaggaggat caagagcggc 120

tctctgtaca tgctgtgcac cggcaacagc agccacagca gctgggataa tcagtgccag 180

tgcaccagca gcgctacaag aaacaccaca aaacaagtga ccccccagcc cgaggagcaa 240

aaggaaagaa agaccaccga aatgcagtcc cccatgcagc ccgtggatca agcctctctg 300

cccggccact gtagagagcc ccctccttgg gaaaatgagg ccacagagag gatctaccac 360

tttgtggtgg gacagatggt ctactaccag tgtgtccaag gctatagagc tctccacaga 420

ggccccgccg agagcgtctg caaaatgaca cacggcaaga caagatggac ccaaccccag 480

ctgatttgta ccggc 495

<210> 122
<211> 495
<212> DNA
<213> Artificial Sequence

<400> 122
gagctctgct gtgatgatcc ccccgaaatc ccccacgcca cattcaaggc catggcctac 60

aaggagggaa ccatgctcaa ctgcgagtgc aagaggggat ttaggaggat caagagcggc 120

tctctgtaca tgctgtgcac cggcaacagc agccacagca gctgggataa tcagtgccag 180

tgcaccagca gcgctacaag aaacaccaca aaacaagtga ccccccagcc cgaggagcaa 240

aaggaaagaa agaccaccga aatgcagtcc cccatgcagc ccgtggatca agcctctctg 300

cccggccact gtagagagcc ccctccttgg gaaaatgagg ccacagagag gatctaccac 360

tttgtggtgg gacagatggt ctactaccag tgtgtccaag gctatagagc tctccacaga 420

ggccccgccg agagcgtctg caaaatgaca cacggcaaga caagatggac ccaaccccag 480

ctgatttgta ccggc 495

<210> 123
<211> 495
<212> DNA
<213> Artificial Sequence

<400> 123
gagctctgcg acgatgatcc ccccgaaatc ccccacgcca cattcaaggc catggcctac 60

aaggagggaa ccatgctcaa ctgcgagtgc aagaggggat ttaggaggat caagagcggc 120

tcttgttaca tgctgtgcac cggcaacagc agccacagca gctgggataa tcagtgccag 180

tgcaccagca gcgctacaag aaacaccaca aaacaagtga ccccccagcc cgaggagcaa 240

aaggaaagaa agaccaccga aatgcagtcc cccatgcagc ccgtggatca agcctctctg 300

cccggccact gtagagagcc ccctccttgg gaaaatgagg ccacagagag gatctaccac 360

tttgtggtgg gacagatggt ctactaccag tgtgtccaag gctatagagc tctccacaga 420

ggccccgccg agagcgtctg caaaatgaca cacggcaaga caagatggac ccaaccccag 480

ctgatttgta ccggc 495

<210>  124
<211>  495
<212>  DNA
<213>  Artificial Sequence

<400>  124
gagctctgcg acgatgatcc ccccgaaatc ccccacgcca cattcaaggc catggcctac      60

aaggagggaa ccatgctcaa ctgcgagtgc aagaggggat tttgtaggat caagagcggc      120

tctctgtaca tgctgtgcac cggcaacagc agccacagca gctgggataa tcagtgccag      180

tgcaccagca gcgctacaag aaacaccaca aaacaagtga ccccccagcc cgaggagcaa      240

aaggaaagaa agaccaccga aatgcagtcc cccatgcagc ccgtggatca agcctctctg      300

cccggccact gtagagagcc ccctccttgg gaaaatgagg ccacagagag gatctaccac      360

tttgtggtgg gacagatggt ctactaccag tgtgtccaag gctatagagc tctccacaga      420

ggccccgccg agagcgtctg caaaatgaca cacggcaaga caagatggac ccaaccccag      480

ctgatttgta ccggc                                                       495


<210>  125
<211>  495
<212>  DNA
<213>  Artificial Sequence

<400>  125
gagctctgcg acgatgatcc ccccgaaatc ccccacgcca cattcaaggc catggcctac      60

aaggagggaa ccatgctcaa ctgcgagtgc aagaggggat ttaggaggat caagagcggc      120

tctctgtaca tgctgtgcac cggcaacagc agccacagca gctgggataa tcagtgccag      180

tgcaccagca gcgctacaag aaacaccaca aaacaagtga ccccccagcc cgaggagcaa      240

aaggaaagaa agaccaccga aatgcagtcc cccatgcagc ccgtggatca agcctctctg      300

cccggccact gtagagagcc ccctccttgg gaaaatgagg ccacagagag gatctactgt      360

tttgtggtgg gacagatggt ctactaccag tgtgtccaag gctatagagc tctccacaga      420

ggccccgccg agagcgtctg caaaatgaca cacggcaaga caagatggac ccaaccccag      480

ctgatttgta ccggc                                                       495


<210>  126
<211>  495
<212>  DNA
<213>  Artificial Sequence

<400>  126
gagctctgcg acgatgatcc ccccgaaatc ccccacgcca cattcaaggc catggcctac      60

aaggagggaa cctgtctcaa ctgcgagtgc aagaggggat ttaggaggat caagagcggc      120

tctctgtaca tgctgtgcac cggcaacagc agccacagca gctgggataa tcagtgccag      180

tgcaccagca gcgctacaag aaacaccaca aaacaagtga ccccccagcc cgaggagcaa 240

aaggaaagaa agaccaccga aatgcagtcc cccatgcagc ccgtggatca agcctctctg 300

cccggccact gtagagagcc ccctccttgg gaaaatgagg ccacagagag gatctaccac 360

tttgtggtgg gacagatggt ctactaccag tgtgtccaag gctatagagc tctccacaga 420

ggccccgccg agagcgtctg caaaatgaca cacggcaaga caagatggac ccaaccccag 480

ctgatttgta ccggc 495

<210> 127
<211> 495
<212> DNA
<213> Artificial Sequence

<400> 127
gagctctgcg acgatgatcc ccccgaaatc ccccacgcca cattcaaggc catggcctac 60

aaggagggaa ccatgctcaa ctgcgagtgc aagaggggat ttaggaggat caagtgtggc 120

tctctgtaca tgctgtgcac cggcaacagc agccacagca gctgggataa tcagtgccag 180

tgcaccagca gcgctacaag aaacaccaca aaacaagtga ccccccagcc cgaggagcaa 240

aaggaaagaa agaccaccga aatgcagtcc cccatgcagc ccgtggatca agcctctctg 300

cccggccact gtagagagcc ccctccttgg gaaaatgagg ccacagagag gatctaccac 360

tttgtggtgg gacagatggt ctactaccag tgtgtccaag gctatagagc tctccacaga 420

ggccccgccg agagcgtctg caaaatgaca cacggcaaga caagatggac ccaaccccag 480

ctgatttgta ccggc 495

<210> 128
<211> 495
<212> DNA
<213> Artificial Sequence

<400> 128
gagctctgcg acgatgatcc ccccgaaatc ccccacgcca cattcaaggc catggcctac 60

aaggagggaa ccatgctcaa ctgcgagtgc aagaggggat ttaggaggat caagagcggc 120

tctctgtaca tgctgtgcac cggcaacagc agccacagca gctgggataa tcagtgccag 180

tgcaccagca gcgctacaag aaacaccaca aaacaagtga ccccccagcc cgaggagcaa 240

aaggaaagaa agaccaccga aatgcagtcc cccatgcagc ccgtggatca agcctctctg 300

cccggccact gtagagagcc ccctccttgg gaaaatgagg ccacagagag gtgttaccac 360

tttgtggtgg gacagatggt ctactaccag tgtgtccaag gctatagagc tctccacaga 420

ggccccgccg agagcgtctg caaaatgaca cacggcaaga caagatggac ccaaccccag 480

ctgatttgta ccggc 495

<210>  129
<211>  495
<212>  DNA
<213>  Artificial Sequence

<400>  129
gagctctgct gtgatgatcc ccccgaaatc ccccacgcca cattcaaggc catggcctac      60

aaggagggaa ccatgctcaa ctgcgagtgc aagaggggat ttaggaggat caagagcggc     120

tctctgtaca tgctgtgcac cggcaacagc agccacagca gctgggataa tcagtgccag     180

tgcaccagca gcgctacaag aaacaccaca aaacaagtga ccccccagcc cgaggagcaa     240

aaggaaagaa agaccaccga aatgcagtcc cccatgcagc ccgtggatca agcctctctg     300

cccggccact gtagagagcc ccctccttgg gaaaatgagg ccacagagag gatctaccac     360

tttgtggtgg gacagatggt ctactaccag tgtgtccaag gctatagagc tctccacaga     420

ggccccgccg agagcgtctg caaaatgaca cacggcaaga caagatggac ccaaccccag     480

ctgatttgta ccggc                                                      495

<210>  130
<211>  495
<212>  DNA
<213>  Artificial Sequence

<400>  130
gagctctgct gtgatgatcc ccccgaaatc ccccacgcca cattcaaggc catggcctac      60

aaggagggaa ccatgctcaa ctgcgagtgc aagaggggat ttaggaggat caagagcggc     120

tctctgtaca tgctgtgcac cggcaacagc agccacagca gctgggataa tcagtgccag     180

tgcaccagca gcgctacaag aaacaccaca aaacaagtga ccccccagcc cgaggagcaa     240

aaggaaagaa agaccaccga aatgcagtcc cccatgcagc ccgtggatca agcctctctg     300

cccggccact gtagagagcc ccctccttgg gaaaatgagg ccacagagag gatctaccac     360

tttgtggtgg gacagatggt ctactaccag tgtgtccaag gctatagagc tctccacaga     420

ggccccgccg agagcgtctg caaaatgaca cacggcaaga caagatggac ccaaccccag     480

ctgatttgta ccggc                                                      495

<210>  131
<211>  495
<212>  DNA
<213>  Artificial Sequence

<400>  131
gagctctgct gtgatgatcc ccccgaaatc ccccacgcca cattcaaggc catggcctac      60

aaggagggaa ccatgctcaa ctgcgagtgc aagaggggat ttaggaggat caagagcggc     120

tctctgtaca tgctgtgcac cggcaacagc agccacagca gctgggataa tcagtgccag     180

tgcaccagca gcgctacaag aaacaccaca aaacaagtga ccccccagcc cgaggagcaa     240

aaggaaagaa agaccaccga aatgcagtcc cccatgcagc ccgtggatca agcctctctg 300

cccggccact gtagagagcc ccctccttgg gaaaatgagg ccacagagag gatctaccac 360

tttgtggtgg gacagatggt ctactaccag tgtgtccaag gctatagagc tctccacaga 420

ggccccgccg agagcgtctg caaaatgaca cacggcaaga caagatggac ccaaccccag 480

ctgatttgta ccggc 495

<210> 132
<211> 495
<212> DNA
<213> Artificial Sequence

<400> 132
gagctctgcg actgtgatcc ccccgaaatc ccccacgcca cattcaaggc catggcctac 60

aaggagggaa ccatgctcaa ctgcgagtgc aagaggggat ttaggaggat caagagcggc 120

tctctgtaca tgctgtgcac cggcaacagc agccacagca gctgggataa tcagtgccag 180

tgcaccagca gcgctacaag aaacaccaca aaacaagtga ccccccagcc cgaggagcaa 240

aaggaaagaa agaccaccga aatgcagtcc cccatgcagc ccgtggatca agcctctctg 300

cccggccact gtagagagcc ccctccttgg gaaaatgagg ccacagagag gatctaccac 360

tttgtggtgg gacagatggt ctactaccag tgtgtccaag gctatagagc tctccacaga 420

ggccccgccg agagcgtctg caaaatgaca cacggcaaga caagatggac ccaaccccag 480

ctgatttgta ccggc 495

<210> 133
<211> 495
<212> DNA
<213> Artificial Sequence

<400> 133
gagctctgcg acgatgatcc ccccgaaatc ccccacgcca cattcaaggc catggcctac 60

aaggagggaa ccatgctcaa ctgcgagtgc aagaggggat ttaggaggat caagagcggc 120

tcttgttaca tgctgtgcac cggcaacagc agccacagca gctgggataa tcagtgccag 180

tgcaccagca gcgctacaag aaacaccaca aaacaagtga ccccccagcc cgaggagcaa 240

aaggaaagaa agaccaccga aatgcagtcc cccatgcagc ccgtggatca agcctctctg 300

cccggccact gtagagagcc ccctccttgg gaaaatgagg ccacagagag gatctaccac 360

tttgtggtgg gacagatggt ctactaccag tgtgtccaag gctatagagc tctccacaga 420

ggccccgccg agagcgtctg caaaatgaca cacggcaaga caagatggac ccaaccccag 480

ctgatttgta ccggc 495

<210> 134

<211>  495
<212>  DNA
<213>  Artificial Sequence

<400>  134
gagctctgcg acgatgatcc ccccgaaatc ccccacgcca cattcaaggc catggcctac      60

aaggagggaa ccatgctcaa ctgcgagtgc aagaggggat ttaggaggat caagagcggc     120

tctctgtaca tgctgtgcac cggcaacagc agccacagca gctgggataa tcagtgccag     180

tgcaccagca gcgctacaag aaacaccaca aaacaagtga ccccccagcc cgaggagcaa     240

aaggaaagaa agaccaccga aatgcagtcc cccatgcagc ccgtggatca agcctctctg     300

cccggccact gtagagagcc ccctccttgg gaaaatgagg ccacagagag gatctgtcac     360

tttgtggtgg gacagatggt ctactaccag tgtgtccaag gctatagagc tctccacaga     420

ggccccgccg agagcgtctg caaaatgaca cacggcaaga caagatggac ccaaccccag     480

ctgatttgta ccggc                                                      495

<210>  135
<211>  495
<212>  DNA
<213>  Artificial Sequence

<400>  135
gagctctgcg acgatgatcc ccccgaaatc ccccacgcca cattcaaggc catggcctac      60

aaggagggaa ccatgctcaa ctgcgagtgc aagaggggat tttgtaggat caagagcggc     120

tctctgtaca tgctgtgcac cggcaacagc agccacagca gctgggataa tcagtgccag     180

tgcaccagca gcgctacaag aaacaccaca aaacaagtga ccccccagcc cgaggagcaa     240

aaggaaagaa agaccaccga aatgcagtcc cccatgcagc ccgtggatca agcctctctg     300

cccggccact gtagagagcc ccctccttgg gaaaatgagg ccacagagag gatctaccac     360

tttgtggtgg gacagatggt ctactaccag tgtgtccaag gctatagagc tctccacaga     420

ggccccgccg agagcgtctg caaaatgaca cacggcaaga caagatggac ccaaccccag     480

ctgatttgta ccggc                                                      495

<210>  136
<211>  495
<212>  DNA
<213>  Artificial Sequence

<400>  136
gagctctgcg acgatgatcc ccccgaaatc ccccacgcca cattcaaggc catggcctac      60

aaggagggaa ccatgctcaa ctgcgagtgc aagaggggat ttaggtgtat caagagcggc     120

tctctgtaca tgctgtgcac cggcaacagc agccacagca gctgggataa tcagtgccag     180

tgcaccagca gcgctacaag aaacaccaca aaacaagtga ccccccagcc cgaggagcaa     240

aaggaaagaa agaccaccga aatgcagtcc cccatgcagc ccgtggatca agcctctctg 300

cccggccact gtagagagcc ccctccttgg gaaaatgagg ccacagagag gatctaccac 360

tttgtggtgg gacagatggt ctactaccag tgtgtccaag gctatagagc tctccacaga 420

ggccccgccg agagcgtctg caaaatgaca cacggcaaga caagatggac ccaaccccag 480

ctgatttgta ccggc 495

<210> 137
<211> 495
<212> DNA
<213> Artificial Sequence

<400> 137
gagctctgcg acgatgatcc ccccgaaatc ccccacgcca cattcaaggc catggcctac 60

aaggagggaa ccatgctcaa ctgcgagtgc aagaggggat ttaggaggat caagagcggc 120

tcttgttaca tgctgtgcac cggcaacagc agccacagca gctgggataa tcagtgccag 180

tgcaccagca gcgctacaag aaacaccaca aaacaagtga ccccccagcc cgaggagcaa 240

aaggaaagaa agaccaccga aatgcagtcc cccatgcagc ccgtggatca agcctctctg 300

cccggccact gtagagagcc ccctccttgg gaaaatgagg ccacagagag gatctaccac 360

tttgtggtgg gacagatggt ctactaccag tgtgtccaag gctatagagc tctccacaga 420

ggccccgccg agagcgtctg caaaatgaca cacggcaaga caagatggac ccaaccccag 480

ctgatttgta ccggc 495

<210> 138
<211> 495
<212> DNA
<213> Artificial Sequence

<400> 138
gagctctgcg acgatgatcc ccccgaaatc ccccacgcca cattcaaggc catggcctac 60

aaggagggaa ccatgctcaa ctgcgagtgc aagaggggat ttaggaggat caagagcggc 120

tcttgttaca tgctgtgcac cggcaacagc agccacagca gctgggataa tcagtgccag 180

tgcaccagca gcgctacaag aaacaccaca aaacaagtga ccccccagcc cgaggagcaa 240

aaggaaagaa agaccaccga aatgcagtcc cccatgcagc ccgtggatca agcctctctg 300

cccggccact gtagagagcc ccctccttgg gaaaatgagg ccacagagag gatctaccac 360

tttgtggtgg gacagatggt ctactaccag tgtgtccaag gctatagagc tctccacaga 420

ggccccgccg agagcgtctg caaaatgaca cacggcaaga caagatggac ccaaccccag 480

ctgatttgta ccggc 495

<210> 139
<211> 495

<212>  DNA
<213>  Artificial Sequence

<400>  139
gagctctgcg acgatgatcc ccccgaaatc ccccacgcca cattcaaggc catggcctac      60

aaggagggaa ccatgctcaa ctgcgagtgc aagaggggat ttaggaggat ctgtagcggc     120

tctctgtaca tgctgtgcac cggcaacagc agccacagca gctgggataa tcagtgccag     180

tgcaccagca gcgctacaag aaacaccaca aaacaagtga ccccccagcc cgaggagcaa     240

aaggaaagaa agaccaccga aatgcagtcc cccatgcagc ccgtggatca agcctctctg     300

cccggccact gtagagagcc ccctccttgg gaaaatgagg ccacagagag gatctaccac     360

tttgtggtgg gacagatggt ctactaccag tgtgtccaag gctatagagc tctccacaga     420

ggccccgccg agagcgtctg caaaatgaca cacggcaaga caagatggac ccaaccccag     480

ctgatttgta ccggc                                                      495


<210>  140
<211>  495
<212>  DNA
<213>  Artificial Sequence

<400>  140
gagctctgcg acgatgatcc ccccgaaatc ccccacgcca cattcaaggc catggcctac      60

aaggagggaa ccatgctcaa ctgcgagtgc aagaggggat ttaggaggat ctgtagcggc     120

tctctgtaca tgctgtgcac cggcaacagc agccacagca gctgggataa tcagtgccag     180

tgcaccagca gcgctacaag aaacaccaca aaacaagtga ccccccagcc cgaggagcaa     240

aaggaaagaa agaccaccga aatgcagtcc cccatgcagc ccgtggatca agcctctctg     300

cccggccact gtagagagcc ccctccttgg gaaaatgagg ccacagagag gatctaccac     360

tttgtggtgg gacagatggt ctactaccag tgtgtccaag gctatagagc tctccacaga     420

ggccccgccg agagcgtctg caaaatgaca cacggcaaga caagatggac ccaaccccag     480

ctgatttgta ccggc                                                      495


<210>  141
<211>  495
<212>  DNA
<213>  Artificial Sequence

<400>  141
gagctctgcg acgatgatcc ccccgaaatc ccccacgcca cattcaaggc catggcctac      60

aaggagggaa ccatgctcaa ctgcgagtgc aagaggggat ttaggaggat caagtgtggc     120

tctctgtaca tgctgtgcac cggcaacagc agccacagca gctgggataa tcagtgccag     180

tgcaccagca gcgctacaag aaacaccaca aaacaagtga ccccccagcc cgaggagcaa     240

aaggaaagaa agaccaccga aatgcagtcc cccatgcagc ccgtggatca agcctctctg     300

cccggccact gtagagagcc ccctccttgg gaaaatgagg ccacagagag gatctaccac 360

tttgtggtgg gacagatggt ctactaccag tgtgtccaag gctatagagc tctccacaga 420

ggccccgccg agagcgtctg caaaatgaca cacggcaaga caagatggac ccaaccccag 480

ctgatttgta ccggc 495

<210> 142
<211> 495
<212> DNA
<213> Artificial Sequence

<400> 142
gagctctgcg acgatgatcc ccccgaaatc ccccacgcca cattcaaggc catggcctac 60

aaggagggaa ccatgctcaa ctgcgagtgc aagaggggat ttaggaggat caagagctgt 120

tctctgtaca tgctgtgcac cggcaacagc agccacagca gctgggataa tcagtgccag 180

tgcaccagca gcgctacaag aaacaccaca aaacaagtga ccccccagcc cgaggagcaa 240

aaggaaagaa agaccaccga aatgcagtcc cccatgcagc ccgtggatca agcctctctg 300

cccggccact gtagagagcc ccctccttgg gaaaatgagg ccacagagag gatctaccac 360

tttgtggtgg gacagatggt ctactaccag tgtgtccaag gctatagagc tctccacaga 420

ggccccgccg agagcgtctg caaaatgaca cacggcaaga caagatggac ccaaccccag 480

ctgatttgta ccggc 495

<210> 143
<211> 495
<212> DNA
<213> Artificial Sequence

<400> 143
gagctctgcg acgatgatcc ccccgaaatc ccccacgcca cattcaaggc catggcctac 60

aaggagggaa ccatgctcaa ctgcgagtgc aagaggggat ttaggaggat caagagcggc 120

tgtctgtaca tgctgtgcac cggcaacagc agccacagca gctgggataa tcagtgccag 180

tgcaccagca gcgctacaag aaacaccaca aaacaagtga ccccccagcc cgaggagcaa 240

aaggaaagaa agaccaccga aatgcagtcc cccatgcagc ccgtggatca agcctctctg 300

cccggccact gtagagagcc ccctccttgg gaaaatgagg ccacagagag gatctaccac 360

tttgtggtgg gacagatggt ctactaccag tgtgtccaag gctatagagc tctccacaga 420

ggccccgccg agagcgtctg caaaatgaca cacggcaaga caagatggac ccaaccccag 480

ctgatttgta ccggc 495

<210> 144
<211> 495
<212> DNA

<213>  Artificial Sequence

<400>  144
gagctctgcg acgatgatcc ccccgaaatc ccccacgcca cattcaaggc catggcctac      60

aaggagggaa ccatgctcaa ctgcgagtgc aagaggggat ttaggaggat ctgtagcggc     120

tctctgtaca tgctgtgcac cggcaacagc agccacagca gctgggataa tcagtgccag     180

tgcaccagca gcgctacaag aaacaccaca aaacaagtga ccccccagcc cgaggagcaa     240

aaggaaagaa agaccaccga aatgcagtcc cccatgcagc ccgtggatca agcctctctg     300

cccggccact gtagagagcc ccctccttgg gaaaatgagg ccacagagag gatctaccac     360

tttgtggtgg gacagatggt ctactaccag tgtgtccaag gctatagagc tctccacaga     420

ggccccgccg agagcgtctg caaaatgaca cacggcaaga caagatggac ccaaccccag     480

ctgatttgta ccggc                                                      495

<210>  145
<211>  495
<212>  DNA
<213>  Artificial Sequence

<400>  145
gagctctgcg acgatgatcc ccccgaaatc ccccacgcca cattcaaggc catggcctac      60

aaggagggaa ccatgctcaa ctgcgagtgc aagaggggat ttaggaggat caagagcggc     120

tctctgtaca tgctgtgcac cggcaacagc agccacagca gctgggataa tcagtgccag     180

tgcaccagca gcgctacaag aaacaccaca aaacaagtga ccccccagcc cgaggagcaa     240

aaggaaagaa agaccaccga aatgcagtcc cccatgcagc ccgtggatca agcctctctg     300

cccggccact gtagagagcc ccctccttgg gaaaatgagg ccacagagag gatctaccac     360

tttgtggtgg gacagatggt ctactaccag tgtgtccaag gctatagagc tctccacaga     420

ggccccgccg agagcgtctg caaaatgaca cacggcaaga caagatggac ccaaccccag     480

ctgatttgta ccggc                                                      495

<210>  146
<211>  399
<212>  DNA
<213>  Artificial Sequence

<400>  146
gcccccacaa gctccagcac caagaagaca cagctgcagc tggaacatct gctgctggac      60

ctccagatga ttctgaacgg catcaacaac tacaagaatc ccaagctgac aagaatgctc     120

accttcaagt tctacatgcc caagaaggcc accgagctga agcatctgca gtgtctggag     180

gaggagctga agcctctgga agaggtgctg aatctggccc agtccaagaa cttccatctg     240

aggcctagag atctgatcag caacatcaac gtcatcgtgc tggagctgaa gggcagcgag     300

accaccttca tgtgcgagta cgccgatgag accgccacca tcgtggagtt tctgaataga 360

tggatcacct tctgccagag catcatcagc acactgacc 399

## Description

### TECHNICAL FIELD

The present invention belongs to the field of molecular biology, and particularly relates to interleukin-2 derivatives and complexes thereof.

### BACKGROUND ART

Interleukin-2 (IL-2), discovered in 1976, was called T cell growth factor (TCGF) at the time. IL-2 is a globular glycoprotein that plays an important role in maintaining the normal functions of T lymphocytes and NK cells. Natural IL-2 is a polypeptide composed of 133 amino acid residues, which has a molecular weight of about 15kD and three cysteine residues, located at positions 58, 105 and 125, respectively. Posttranslational modifications include Thr glycosylation at position 3, disulfide bonds formed by cysteine residues at positions 58 and 105, and the formation of high-level structures mainly composed of 4 α helices and some linking sequences (loops), which are essential for IL-2's function (Bazan et al., Science 257, 410-413 (1992)).

IL-2 is produced mainly by activated T cells and is capable of: promoting the proliferation and differentiation of T cells to maintain their activity; stimulating the production, proliferation and activation of natural killer (NK) cells; inducing the generation of cytotoxic T lymphocytes (CTLs); inducing and activating lymphokineactivated cell killer (LAK) cells and tumor infiltrating lymphocytes; promoting the expression of cytokines and cytolytic molecules by T cells; and promoting the proliferation of B cells (Waldmann et al., Nat. Rev. Immunol. 6,595-601 (2009)). All of these cells directly or indirectly have the effect of killing cells infected by the foreign microorganisms and cancerous cells. Therefore, IL-2 has good antiviral and anticancer effects and wide clinical application potential.

IL-2 mediates its action by binding to IL-2 receptor (IL-2R), which consists of 3 subunits, namely α (CD25), β (CD122) and γ (CD132) receptor subunits, wherein α receptors are mainly expressed on the surface of T suppressor cells (Treg) and some endothelial cells, while β and γ receptor subunits are highly expressed on effector T cells (Teff) and NK cells. IL-2 differs in its affinity to different complexes of the receptor subunits. IL-2 has high affinity to the complex composed of α, β and γ receptor subunits and intermediate affinity (approximately 100-fold lower) to the complex composed of β and γ receptor subunits. Both of the complexes are able to transmit signal upon IL-2 binding(Minami et al., Annu. Rev. Immunol. 11, 245-268 (1993)). However, clinically, under the condition of low dose of IL-2, it will preferentially bind to the high-affinity receptors on the surface of Treg cells, which will cause immunosuppression and fail to achieve the therapeutic effect. A high dose of IL-2 will neutralize the immune suppression caused by Treg activation by activating a large number of effector T cells. At the same time, there will be more toxic side effects and cell apoptosis.

Due to the anti-tumor effect of IL-2, a high dose of IL-2 (Aldesleukin) was approved by FDA in 1992 for the clinical treatment of melanoma and renal cell carcinoma. However, patients receiving high-dose IL-2 treatment often experience severe side effects, including cardiovascular, pulmonary edematous, hepatic, gastrointestinal, neurological, and hematological events. Most of these side effects can be explained by Vascular (or Capillary) Leak Syndrome (VLS), which is also an indicator for clinical and animal experiments to evaluate the side effects of IL-2 treatment. VLS is caused by the expression of high-affinity receptors (α, β and γ subunits) of IL-2 on endothelial cells (Krieg et al., Proc. Nat. Acad. Sci. USA107, 11906-11 (2010)). Therefore, weakening or elimination of the binding to α receptor will help to weaken the function of IL-2 of promoting the inhibition of T cell proliferation activity, meanwhile which will also reduce the binding to α receptor of endothelial cell, thereby reducing or eliminating the side effects caused by IL-2 treatment. The binding sites of IL-2 and α receptor subunit are mainly at positions 37, 38, 41, 42, 43, 44, 45, 61, 62, 65, 68 and 72 (Rickert. M. et al., Science 308 :1477-1480 (2005)). Merck, Roche or other scientific research institutions have made some mutations around these amino acids on the surface of IL-2 that bind to α receptor subunit. For example, the interaction between the mutant from Merck (R38W, F42K, WO2008003473A2) and α receptor subunit is reduced to activate effector T cells to enhance efficacy. The IL-2 mutants from Roche (F42A, Y45A and L72G, US 2016/0208017A1) do not bind to α receptor, but can normally bind to β and γ receptor subunit complexes, which can exert effects and are currently in clinical practice.

Therefore, the reduction or elimination of the interaction between IL-2 and α receptor subunit may be an important aspect to improve the effectiveness of treatment and to reduce the side effects of treatment in tumor patients.

### SUMMARY OF THE INVENTION

In view of the defects in the prior art, the present invention provides an IL-2 derivative and a complex thereof.

In the first aspect, the present invention provides an IL-2 derivative. In a specific embodiment, the IL-2 derivative is introduced with at least one cysteine residue based on wild-type IL-2, and the binding plane of the IL-2 derivative and α receptor subunit is partially or completely blocked, while the affinity of the IL-2 derivative to the complex of β and γ receptor subunits is basically retained. The amino acid sequence of the wild-type IL-2 is shown in SEQ ID NO: 1.

Further, the at least one cysteine residue introduced based on wild-type IL-2 is capable of:
a) forming an intramolecular disulfide bond of the IL-2 derivative; or
b) enabling the IL-2 derivative to bind to a blocking module through a intermolecular disulfide bond.

Optionally, the at least one cysteine residue is introduced by means of point mutation.

Optionally, in one embodiment, a first cysteine residue and a second cysteine residue are introduced by means of point mutation based on wild-type IL-2; and one or both of the first cysteine residue and the second cysteine residue are amino acids related to the binding plane of the wild-type IL-2 and α receptor subunit, or amino acids in the vicinity thereof. Further, the amino acid positions related to the binding plane of wild-type IL-2 and α receptor subunit are position 37, position 38, position 41, position 42, position 43, position 44, position 45, position 61, position 62, position 65, position 68 and position 72.

Further, the first cysteine residue is an amino acid at position 37, position 38, position 41, position 42, position 43, position 44, position 45, position 61 or position 62 of the wild-type IL-2, or an amino acid in the vicinity thereof; and the second cysteine residue is an amino acid at position 61, position 62, position 65, position 68 or position 72 of the wild-type IL-2, or an amino acid in the vicinity thereof. Optionally, the term "vicinity" refers to: 1) 1 to 4 amino acids that are adjacent in the primary structure; and/or 2) amino acids that are adjacent in the tertiary structure.

Further, the first cysteine residue is an amino acid point mutant selected from the group consisting of: K35C, L36C, R38C, M39C, L40C, T41C, F42C, K43C, F44C and E61C.

Further, the second cysteine residue is an amino acid point mutant selected from the group consisting of: V69C, E62C, P65C, T111C, Y107C, A112C, T113C, 1114C, L72C and A73C.

Optionally, a combination of the first cysteine residue and the second cysteine residue that form the intramolecular disulfide bond is a combination of amino acid point mutants selected from the group consisting of: M39C and V69C; F44C and E62C; F44C and P65C; F42C and V69C; E61C and Y107C; F42C and P65C; F42C and T111C; F42C and A112C; F42C and T113C; T41C and A112C; L40C and A112C; T113C and T113C; L40C and 1114C; M39C and L72C; M39C and A73C; R38C and V69C; R38C and L72C; L36C and V69C; L36C and L72C; L36C and A73C; K35C and V69C; and K43C and A112C.

Optionally, the center-of-mass vector distance between the first cysteine residue and the second cysteine residue is less than 6Å.

Optionally, in a second embodiment, a third cysteine residue is introduced by means of point mutation based on wild-type IL-2; and the third cysteine residue is an amino acid related to the binding plane of wild-type IL-2 and α receptor subunit, or an amino acid in the vicinity thereof.

Further, the third cysteine residue is an amino acid at position 37, position 38, position 41, position 42, position 43, position 44, position 45, position 61, position 62, position 65, position 68 or position 72 of the wild-type IL-2, or an amino acid in the vicinity thereof. Optionally, the term "vicinity" refers to: 1) 1 to 4 amino acids that are adjacent in the primary structure; and/or 2) amino acids that are adjacent in the tertiary structure.

Further, the third cysteine residue is an amino acid point mutant selected from the group consisting of: P34C, K35C, T37C, R38C, T41C, K43C, F44C, Y45C, E61C, E62C, K64C, P65C, E68C and L72C.

Further, the blocking module has or is introduced with a fourth cysteine residue; and the third cysteine residue on the IL-2 derivative and the fourth cysteine residue on the blocking module are capable of forming an intermolecular disulfide bond.

Optionally, the center-of-mass vector distance between the third cysteine residue and the fourth cysteine residue is less than 6Å.

Optionally, the blocking module is an extracellular segment of the α receptor subunit.

Further, the amino acid sequence of the extracellular segment of the α receptor subunit is shown in SEQ ID NO: 24.

Further, the fourth cysteine residue on the extracellular segment of the α receptor subunit is an amino acid point mutant selected from the group consisting of: D4C, D5C, M25C, N27C, R35C, R36C, K38C, S39C, G40C, S41C, L42C, I118C, Y119C and H120C.

Optionally, a combination of the third cysteine residue and the fourth cysteine residue that form the intramolecular disulfide bond is a combination of amino acid point mutants selected from the group consisting of: T41C and N27C; P34C and D4C; E68C and L42C; Y45C and R35C; R38C and H120C; L72C and M25C; E61C and S39C; T41C and I118C; K35C and D4C; T37C and D4C; R38C and D4C; R38C and D5C; T41C and L42C; T41C and Y119C; K43C and R35C; K43C and R36C; F44C and L42C; K43C and L42C; E61C and K38C; E62C and K38C; K64C and S39C; K64C and G40C; K64C and S41C; and P65C and K38C.

In either the first embodiment or the second embodiment, optionally, the cysteine residue at position 125 of the wild-type IL-2 is converted into another amino acid residue by means of point mutation.

Further optionally, the point mutant at position 125 of the wild-type IL-2 is C125A.

Optionally, the amino acid sequence of the IL-2 derivative is shown in SEQ ID NO: 3 to SEQ ID NO: 24, or in SEQ ID NO: 26 to SEQ ID NO: 40.

In the second aspect, the present invention provides a complex. In a specific embodiment, the complex comprises:
1) an IL-2 derivative, which is introduced with a third cysteine residue based on wild-type IL-2; and
2) a blocking module, which has or is introduced with a fourth cysteine residue; wherein the third cysteine residue on the IL-2 derivative and the fourth cysteine residue on the blocking module are capable of forming an intermolecular disulfide bond, thereby forming a complex of the IL-2 derivative and the blocking module; and wherein the binding plane of the IL-2 derivative and α receptor subunit is partially or completely blocked, while the affinity of the IL-2 derivative to the complex of β and γ receptor subunits is basically retained.

Optionally, the at least one cysteine residue is introduced by means of point mutation.

Further, the third cysteine residue is an amino acid related to the binding plane of the wild-type IL-2 and α receptor subunit, or an amino acid in the vicinity thereof.

Further, the third cysteine residue is an amino acid at position 37, position 38, position 41, position 42, position 43, position 44, position 45, position 61, position 62, position 65, position 68 or position 72 of the wild-type IL-2, or an amino acid in the vicinity thereof. Optionally, the term "vicinity" refers to: 1) 1 to 4 amino acids that are adjacent in the primary structure; and/or 2) amino acids that are adjacent in the tertiary structure.

Optionally, the center-of-mass vector distance between the third cysteine residue and the fourth cysteine residue is less than 6Å.

Further, the third cysteine residue is an amino acid point mutant selected from the group consisting of: P34C, K35C, T37C, R38C, T41C, K43C, F44C, Y45C, E61C, E62C, K64C, P65C, E68C and L72C.

Optionally, the blocking module is an extracellular segment of the α receptor subunit, the amino acid sequence of which is shown in SEQ ID NO: 25.

Further, the fourth cysteine residue on the extracellular segment of the α receptor subunit is an amino acid point mutant selected from the group consisting of: D4C, D5C, M25C, N27C, R35C, R36C, K38C, S39C, G40C, S41C, L42C, I118C, Y119C and H120C.

Optionally, a combination of the third cysteine residue and the fourth cysteine residue that form the intramolecular disulfide bond is a combination of amino acid point mutants selected from the group consisting of: T41C and N27C; P34C and D4C; E68C and L42C; Y45C and R35C; R38C and H120C; L72C and M25C; E61C and S39C; T41C and I118C; K35C and D4C; T37C and D4C; R38C and D4C; R38C and D5C; T41C and L42C; T41C and Y119C; K43C and R35C; K43C and R36C; F44C and L42C; K43C and L42C; E61C and K38C; E62C and K38C; K64C and S39C; K64C and G40C; K64C and S41C; and P65C and K38C.

Optionally, the point mutant at position 125 of the wild-type IL-2 is C125A.

Optionally, a combination of the amino acid sequence of the IL-2 derivative and the sequence of the extracellular segment of the α receptor subunit is a combination selected from the group consisting of: SEQ ID NO: 26 and SEQ ID NO: 50; SEQ ID NO: 27 and SEQ ID NO: 51; SEQ ID NO: 28 and SEQ ID NO: 52; SEQ ID NO: 29 and SEQ ID NO: 53; SEQ ID NO: 30 and SEQ ID NO: 54; SEQ ID NO: 31 and SEQ ID NO: 55; SEQ ID NO: 32 and SEQ ID NO: 56; SEQ ID NO: 33 and SEQ ID NO: 57; SEQ ID NO: 34 and SEQ ID NO: 58; SEQ ID NO: 35 and SEQ ID NO: 59; SEQ ID NO: 36 and SEQ ID NO: 60; SEQ ID NO: 37 and SEQ ID NO: 61; SEQ ID NO: 38 and SEQ ID NO: 62; SEQ ID NO: 39 and SEQ ID NO: 63; SEQ ID NO: 40 and SEQ ID NO: 64; SEQ ID NO: 41 and SEQ ID NO: 65; SEQ ID NO: 42 and SEQ ID NO: 66; SEQ ID NO: 43 and SEQ ID NO: 67; SEQ ID NO: 44 and SEQ ID NO: 68; SEQ ID NO: 45 and SEQ ID NO: 69; SEQ ID NO: 46 and SEQ ID NO: 70; SEQ ID NO: 47 and SEQ ID NO: 71; SEQ ID NO: 48 and SEQ ID NO: 72; and SEQ ID NO: 49 and SEQ ID NO: 73.

In the third aspect, the present invention provides an isolated polynucleotide. In a specific embodiment, the isolated polynucleotide encodes the IL-2 derivative as described above or the complex as described above.

In the fourth aspect, the present invention provides an expression vector. In a specific embodiment, the expression vector comprises the isolated polynucleotide as described above.

In the fifth aspect, the present invention provides a host cell. In a specific embodiment, the host cell comprises the isolated polynucleotide as described above.

In the sixth aspect, the present invention provides a composition. In a specific embodiment, the composition comprises the IL-2 derivative as described above or the complex as described above, and a pharmaceutically acceptable carrier.

In the seventh aspect, the present invention provides the use of the IL-2 derivative as described above or the complex as described above for preparing drugs or preparations for treating diseases.

In the eighth aspect, the present invention provides the use of the IL-2 derivative as described above or the complex as described above for preparing a composition for stimulating the immune system of an individual.

In the ninth aspect, the present invention provides a method for producing the IL-2 derivative. In a specific embodiment, the method includes culturing the host cell as described above under conditions suitable for expressing the IL-2 derivative.

In the tenth aspect, the present invention provides a method for producing the complex. In a specific embodiment, the method includes culturing the host cell as described above under conditions suitable for expressing the complex.

In the IL-2 derivative or complex according to specific embodiments of the present invention, by forming intra-molecular or intermolecular disulfide bonds, the binding sites where the IL-2 derivative binds to α receptor are blocked, thereby the structure for binding to the α receptor is precluded.

The IL-2 derivative or complex according to the present invention provides a new direction for reducing VLS, or reducing or eliminating the toxic and side effects caused by IL-2 treatment.

### DESCRIPTION OF THE DRAWINGS

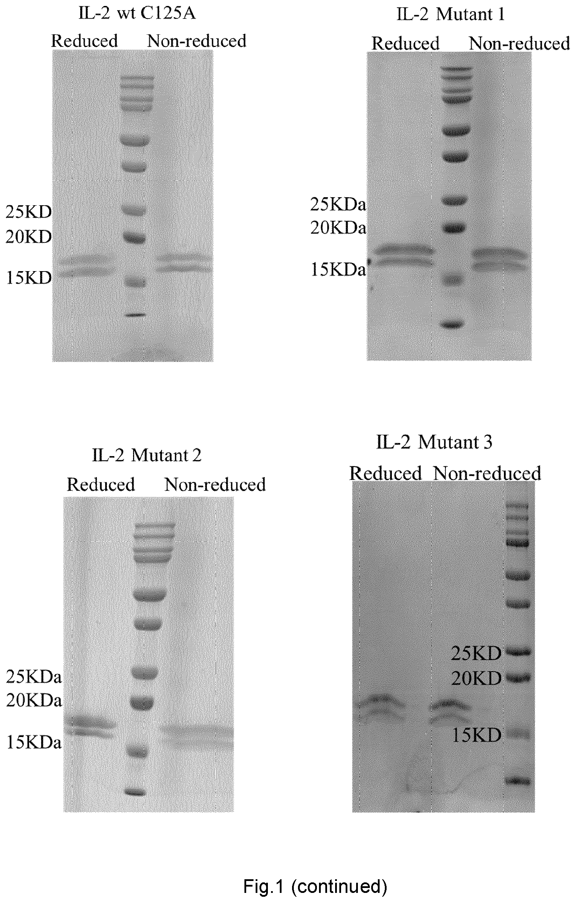

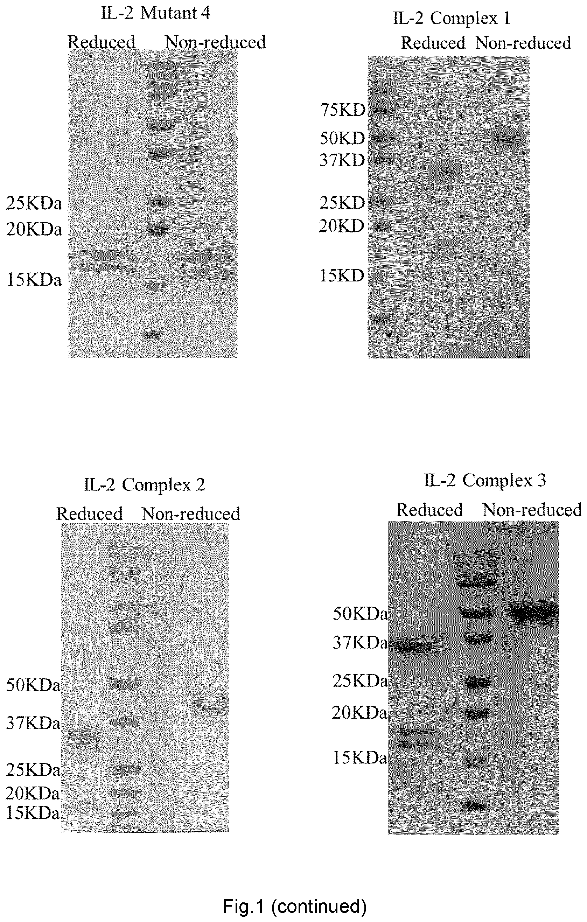

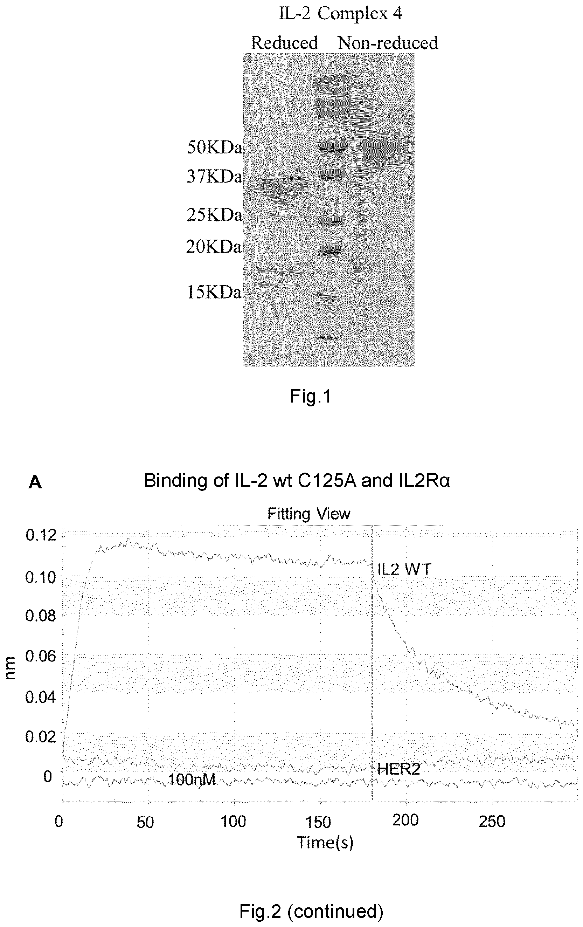

Fig. 1 shows SDS-PAGE electrophoretograms of the purified IL-2 derivatives according to one embodiment of the present invention; wherein the "reduced" means that the reducing agent β-ME was added to the loading buffer, and the "non-reduced" means that no reducing agent was added.

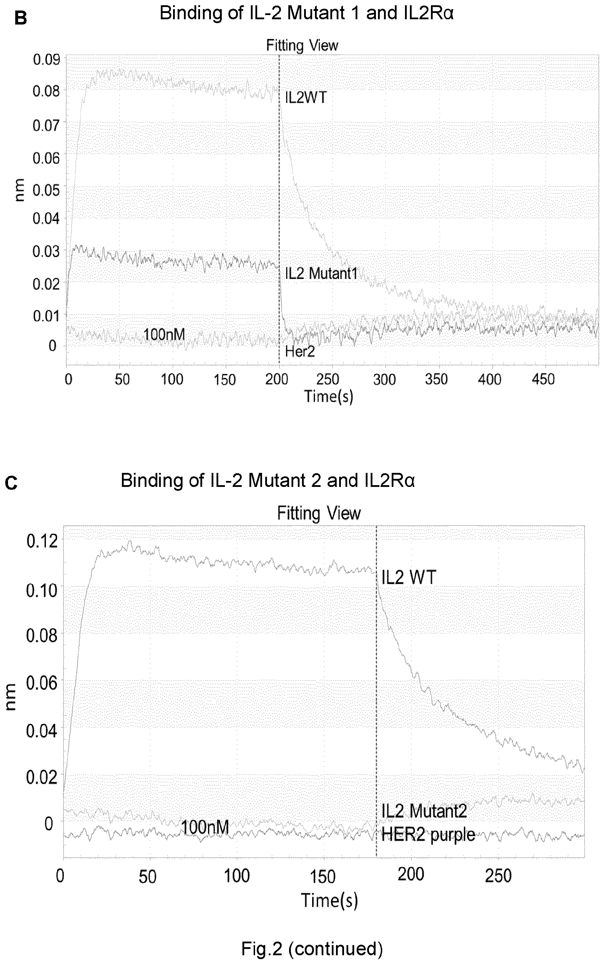

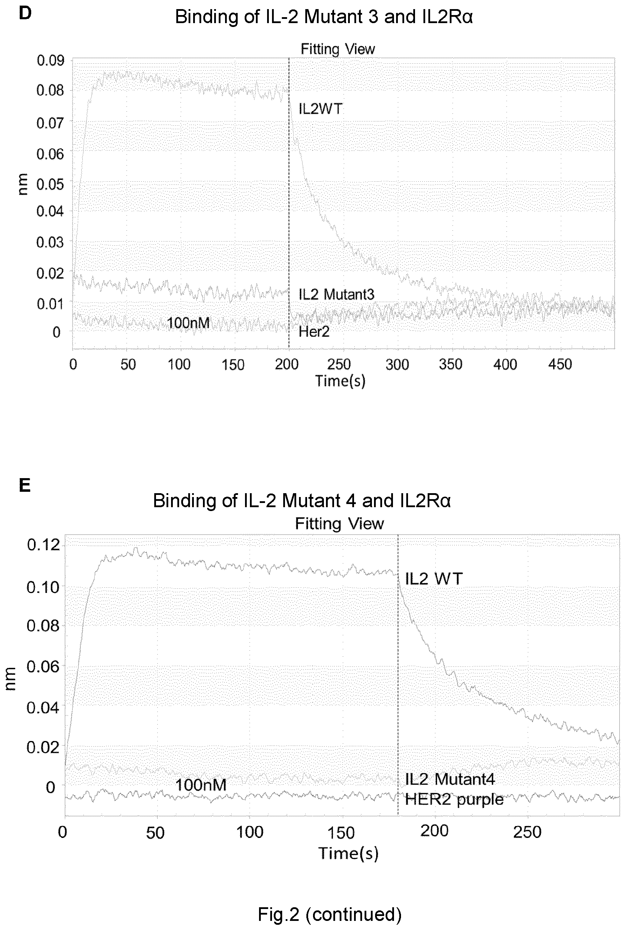

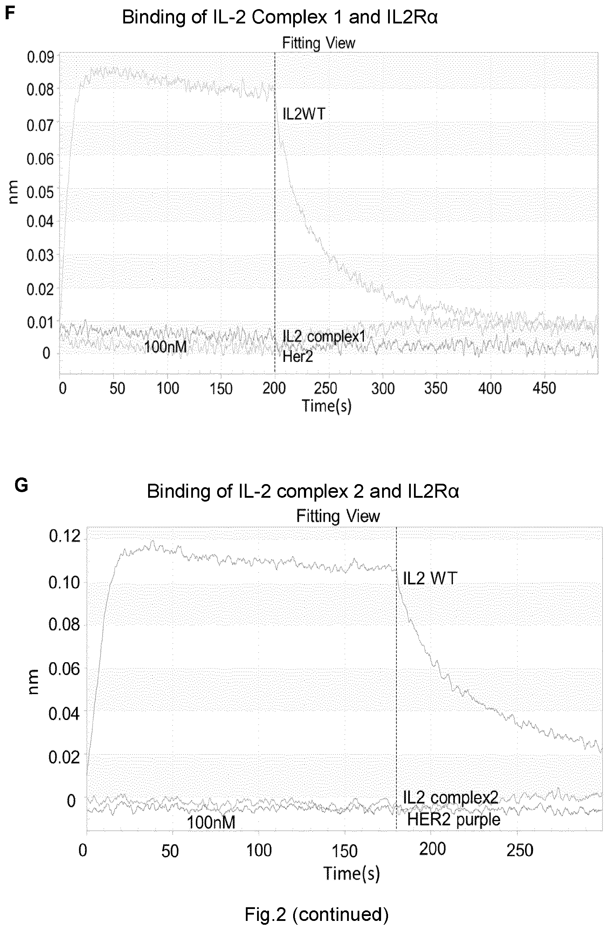

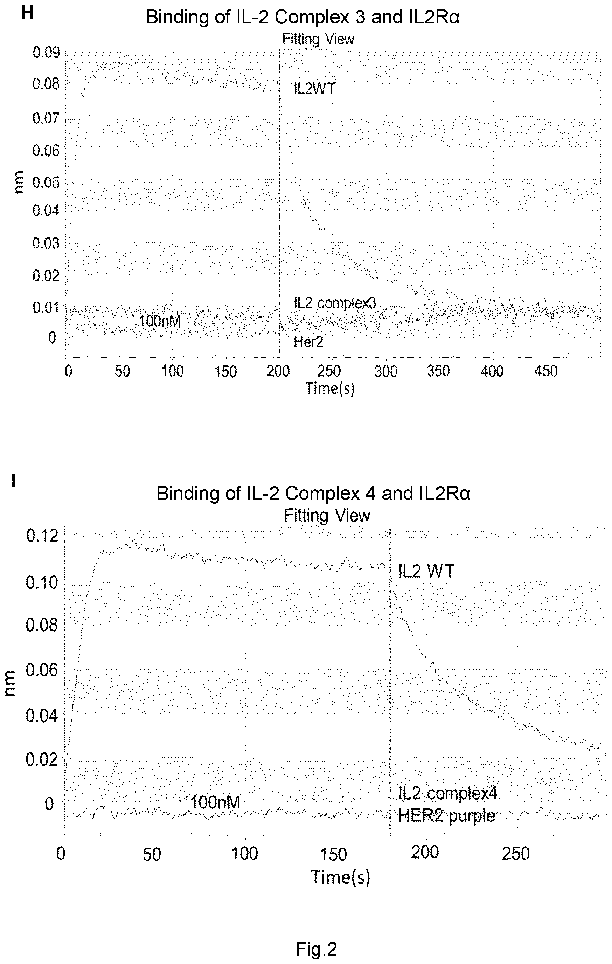

Fig. 2 shows signal graphs of binding affinities of the IL-2 derivatives to IL2Ra tested by Fortebio according to one embodiment of the present invention, wherein the concentration is 100 nM. The irrelevant protein control used is HER2. Among them, Fig. 2Ais related to IL-2 wt C125A; Fig. 2B is related to IL-2 mutant 1; Fig. 2C is related to IL-2 mutant 2; Fig. 2D is related to IL-2 mutant 3; Fig. 2E is related to IL-2 mutant 4; Fig. 2F is related to IL-2 complex 1; Fig. 2G is related to IL-2 complex 2; Fig. 2H is related to IL-2 complex 3; and Fig. 2l is related to IL-2 complex 4.

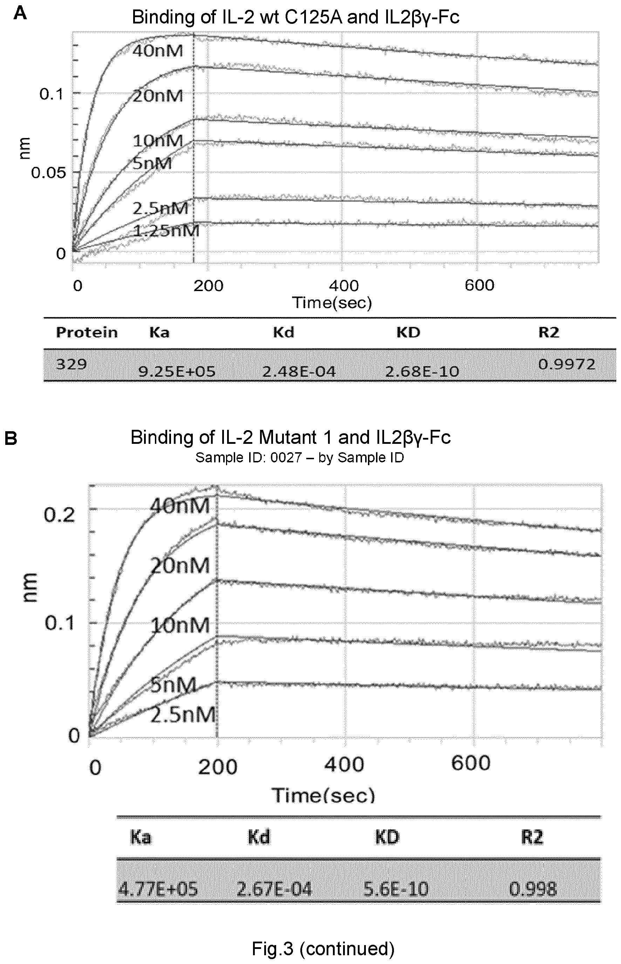

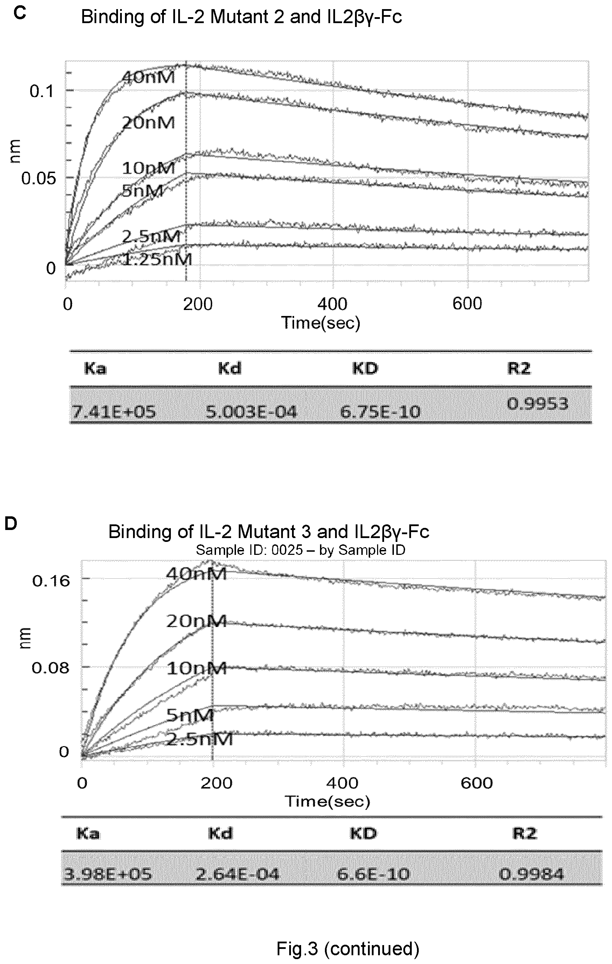

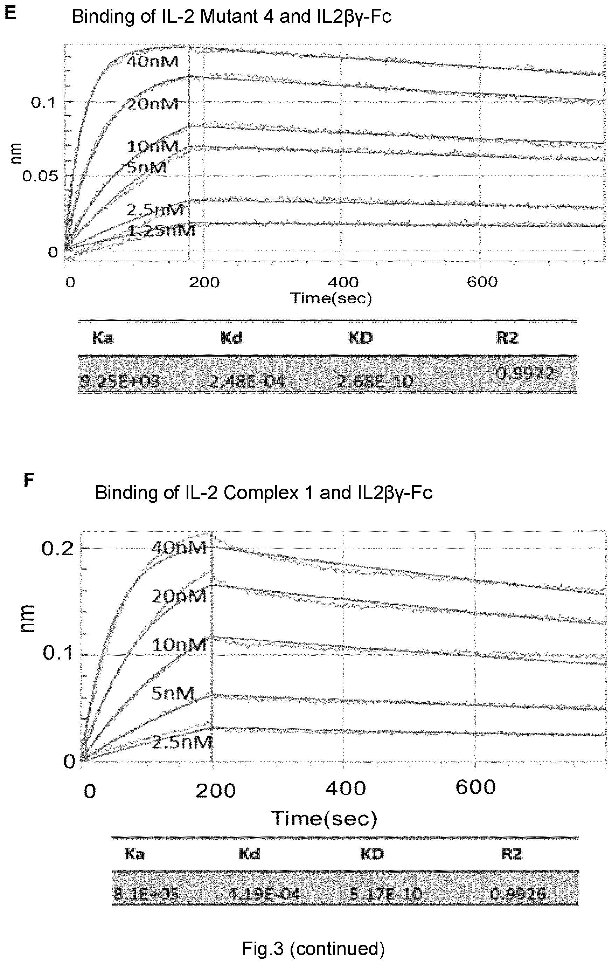

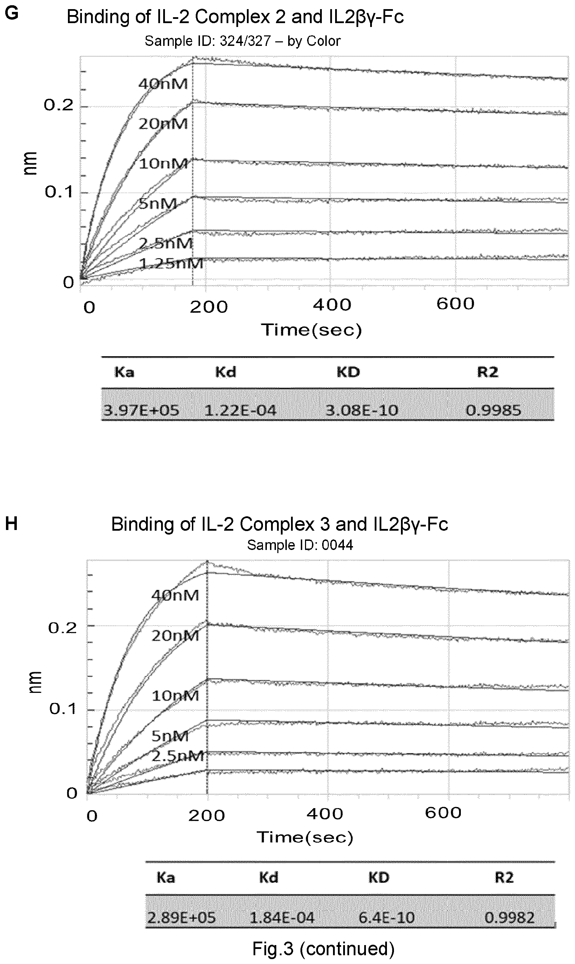

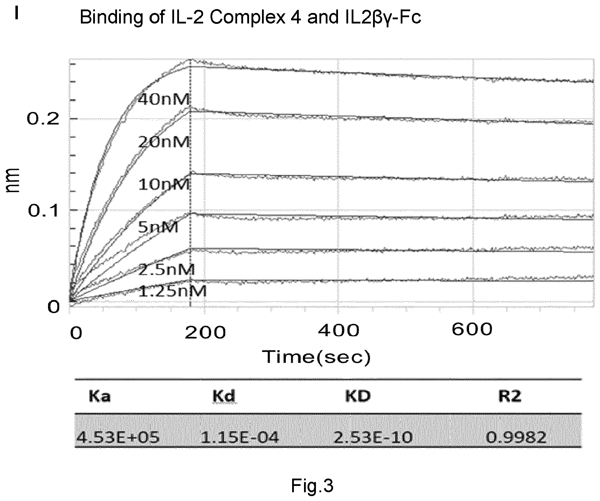

Fig. 3 shows signal graphs of binding affinities of the IL-2 derivatives to IL2Rβγ tested by Fortebio and Ka, Kd, KD and R2 thereof according to one embodiment of the present invention, wherein the concentration is 1.25 nM to 40 nM. Among them, Fig. 3A is related to IL-2 wt C125A; Fig. 3B is related to IL-2 mutant 1; Fig. 3C is related to IL-2 mutant 2; Fig. 3D is related to IL-2 mutant 3; Fig. 3E is related to IL-2 mutant 4; Fig. 3F is related to IL-2 complex 1; Fig. 3G is related to IL-2 complex 2; Fig. 3H is related to IL-2 complex 3; and Fig. 3l is related to IL-2 complex 4.

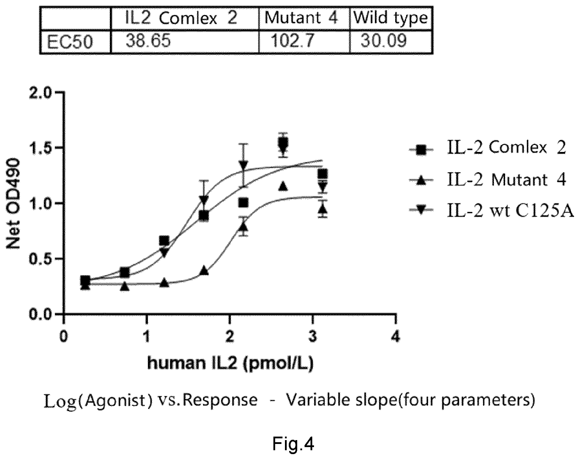

Fig. 4 is a graph illustrating the CTLL-2 (T cell) proliferation experiment according to one embodiment of the present invention.

### DETAILED DESCRIPTION

Hereinafter, the present invention will be further described in conjunction with examples. It should be understood that these examples are used for illustrative purposes only and are not intended to limit the protection scope of the present invention.

In the following examples, the experimental methods without special instructions were usually carried out in accordance with conventional conditions or in accordance with the conditions recommended by the manufacturer. See, for example, Sambrook et al, Molecular Cloning: A Laboratory Manual (New York: Cold Spring Harbor Laboratory Press, 1989). Unless otherwise specified, the reagents used are commercially available or publicly available reagents.

Herein, the relative amino acid positions of the IL-2 derivatives and wild-type IL-2 were determined based on the amino acid sequence of wild-type IL-2 (e.g., SEQ ID NO: 1).

The amino acid sequence of Wild type IL-2 (IL-2 wt, SEQ ID NO: 1) is as follows:



.

The nucleotide sequence of wild-type IL-2 is shown in SEQ ID NO: 146.

Herein, the relative amino acid positions of CD25-ECD were determined based on the amino acid sequence as shown in SEQ ID NO: 25.

Herein, the terms "first", "second", "third", etc., are only used for distinguishing purposes, and are not intended to limit the order. For example, the derivative may only have a "third" cysteine residue, without "first" and "second" cysteine residues.

Herein, the term "IL-2 derivative" encompasses IL-2 mutant, and complex formed by IL-2 mutant with other molecule. The term "IL-2 mutant" refers to a molecule formed through mutations (e.g., point mutations or insertion mutations) based on the wild-type IL-2.

Herein, the term "IL2Rα refers to interleukin-2 receptor α,also referred to as "α receptor subunit"; "IL2Rβ" refers to interleukin-2 receptor β, also referred to as "β receptor subunit"; IL2Rγ" refers to interleukin-2 receptor γ, also referred to as "γ receptor subunit"; and "IL2Rβγ" refers to the complex formed by interleukin-2 receptor β and receptor γ, also referred to as "complex of β and γ receptor subunits".

In the prior art, in order to reduce or eliminate the binding of IL-2 and α receptors, simple mutations are made to the amino acids on the binding plane of IL-2 to α receptor.

In specific embodiments of the present invention, the strategy adopted was to introduce additional cysteine residues to form new disulfide bonds within the IL-2 molecule, or to form complexes of IL-2 and other blocking modules through intermolecular disulfide bonds, which partially or completely blocks the binding sites on IL-2 for α receptor, while the affinity to the complex of β and γ receptor subunits is not affected.

In the first specific embodiment, a first cysteine residue and a second cysteine residue are introduced to form an IL-2 intramolecular disulfide bond, which makes the IL-2 derivative more stable in structure and can further form a barrier to destroy the binding plane for α receptor's binding.

The first cysteine residue and the second cysteine residue are introduced by making appropriate point mutations based on wild-type IL-2. The positions of the first cysteine residue and the second cysteine residue to be introduced are mainly determined by the following methods:
1) The positions of the first and the second cysteine residues were the amino acid residues on the binding plane of IL-2 and α receptor or amino acid residues in the vicinity thereof, wherein the amino acid residues on the binding plane of IL-2 and α receptors were the amino acids at positions 37, 38, 41, 42, 43, 44, 45, 61, 62, 65, 68 and 72;
2) The structure of IL-2 and the distance between atoms should be fully considered; and
3) Through design according to bioinformatics and protein engineering, two suitable sites around the above amino acid residues were found on the basis of the center-of-mass vector distance of the residues, where an intramolecular disulfide bond can be formed, but an intermolecular disulfide bond was difficult or impossible to be formed.

After the suitable sites are determined, the original amino acid residues are mutated into the first cysteine residue and the second cysteine residue by mutation. Then after a normal process of transcription and translation, the mutated IL-2 derivative (IL-2 mutant) was produced with the intramolecular disulfide bond.

In some embodiments, the original free cysteine at position 125 of IL-2 was mutated to prevent its interference to the formation of the intermolecular disulfide bond.

In some embodiments, the amino acid sequences of IL-2 mutants obtained through the design are shown in Table 1.

**Table 1. The amino acid sequence of IL-2 mutants obtained through the design**

| **Mutan t** | **Mutation site** | **Amino acid sequence** | **SEQ ID NO:** | **Nucleotide sequence (SEQ ID NO:)** |
|---|---|---|---|---|
| IL-2 wt (C125 A) | IL-2 wt (C125A) | APTSSSTKKTQLQLEHLLLDLQMILN GINNYKNPKLTRMLTFKFYMPKKATE LKHLQCLEEELKPLEEVLNLAQSKNF HLRPRDLISNINVIVLELKGSETTFMC EYADETATIVEFLNRWITFAQSIISTLT | 2 | 74 |
| IL-2 Mutan t 1 | IL-2 (M39C, V69C, C125A) | APTSSSTKKTQLQLEHLLLDLQMILN GINNYKNPKLTRCLTFKFYMPKKATE LKHLQCLEEELKPLEECLNLAQSKNF HLRPRDLISNINVIVLELKGSETTFMC EYADETATIVEFLNRWITFAQSIISTLT | 3 | 75 |
| IL-2 Mutan t 2 | IL-2 (F44C, E62C, C125A) | APTSSSTKKTQLQLEHLLLDLQMILN GINNYKNPKLTRMLTFKCYMPKKAT ELKHLQCLEECLKPLEEVLNLAQSKN FHLRPRDLISNINVIVLELKGSETTFM CEYADETATIVEFLNRWITFAQSIIST LT | 4 | 76 |
| IL-2 Mutan t 3 | IL-2 (F44C, P65C, C125A) | APTSSSTKKTQLQLEHLLLDLQMILN GINNYKNPKLTRMLTFKCYMPKKAT ELKHLQCLEEELKCLEEVLNLAQSKN FHLRPRDLISNINVIVLELKGSETTFM CEYADETATIVEFLNRWITFAQSIIST LT | 5 | 77 |
| IL-2 Mutan t 4 | IL-2 (F42C, V69C, C125A) | APTSSSTKKTQLQLEHLLLDLQMILN GINNYKNPKLTRMLTCKFYMPKKAT ELKHLQCLEEELKPLEECLNLAQSKN FHLRPRDLISNINVIVLELKGSETTFM CEYADETATIVEFLNRWITFAQSIIST LT | 6 | 78 |
| IL-2 Mutan t 5 | IL-2 (E61 C, Y107C, C125A) | APTSSSTKKTQLQLEHLLLDLQMILN GINNYKNPKLTRMLTFKFYMPKKATE LKHLQCLECELKPLEEVLNLAQSKNF HLRPRDLISNINVIVLELKGSETTFMC ECADETATIVEFLNRWITFAQSIISTL T | 7 | 79 |
| IL-2 Mutan t 6 | IL-2 (F42C, P65C, C125A) | APTSSSTKKTQLQLEHLLLDLQMILN GINNYKNPKLTRMLTCKFYMPKKAT ELKHLQCLEEELKCLEEVLNLAQSKN FHLRPRDLISNINVIVLELKGSETTFM CEYADETATIVEFLNRWITFAQSIIST LT | 8 | 80 |
| IL-2 Mutan t 7 | IL-2 (F42C, T111C, C125A) | APTSSSTKKTQLQLEHLLLDLQMILN GINNYKNPKLTRMLTCKFYMPKKAT ELKHLQCLEEELKPLEEVLNLAQSKN FHLRPRDLISNINVIVLELKGSETTFM CEYADECATIVEFLNRWITFAQSIIST LT | 9 | 81 |
| IL-2 Mutan t 8 | IL-2 (F42C, A112C, C125A) | APTSSSTKKTQLQLEHLLLDLQMILN GINNYKNPKLTRMLTCKFYMPKKAT ELKHLQCLEEELKPLEEVLNLAQSKN FHLRPRDLISNINVIVLELKGSETTFM CEYADETCTIVEFLNRWITFAQSIIST LT | 10 | 82 |
| IL-2 Mutan t 9 | IL-2 (F42C, T113C, C125A) | APTSSSTKKTQLQLEHLLLDLQMILN GINNYKNPKLTRMLTCKFYMPKKAT ELKHLQCLEEELKPLEEVLNLAQSKN FHLRPRDLISNINVIVLELKGSETTFM CEYADETACIVEFLNRWITFAQSIIST LT | 11 | 83 |
| IL-2 Mutan t 10 | IL-2 (T41C, A112C, C125A) | APTSSSTKKTQLQLEHLLLDLQMILN GINNYKNPKLTRMLCFKFYMPKKAT ELKHLQCLEEELKPLEEVLNLAQSKN FHLRPRDLISNINVIVLELKGSETTFM CEYADETCTIVEFLNRWITFAQSIIST LT | 12 | 84 |
| IL-2 Mutan t 11 | IL-2 (L40C, A112C, C125A) | APTSSSTKKTQLQLEHLLLDLQMILN GINNYKNPKLTRMCTFKFYMPKKAT ELKHLQCLEEELKPLEEVLNLAQSKN FHLRPRDLISNINVIVLELKGSETTFM CEYADETCTIVEFLNRWITFAQSIIST LT | 13 | 85 |
| IL-2 Mutan t 12 | IL-2 (L40C, T113C, C125A) | APTSSSTKKTQLQLEHLLLDLQMILN GINNYKNPKLTRMCTFKFYMPKKAT ELKHLQCLEEELKPLEEVLNLAQSKN FHLRPRDLISNINVIVLELKGSETTFM CEYADETACIVEFLNRWITFAQSIIST LT | 14 | 86 |
| IL-2 Mutan t 13 | IL-2 (L40C, 1114C, C125A) | APTSSSTKKTQLQLEHLLLDLQMILN GINNYKNPKLTRMCTFKFYMPKKAT ELKHLQCLEEELKPLEEVLNLAQSKN FHLRPRDLISNINVIVLELKGSETTFM CEYADETATCVEFLNRWITFAQSIIST LT | 15 | 87 |
| IL-2 Mutan t 14 | IL-2 (M39C, L72C, C125A) | APTSSSTKKTQLQLEHLLLDLQMILN GINNYKNPKLTRCLTFKFYMPKKATE LKHLQCLEEELKPLEEVLNCAQSKNF HLRPRDLISNINVIVLELKGSETTFMC EYADETATIVEFLNRWITFAQSIISTLT | 16 | 88 |
| IL-2 Mutan t 15 | IL-2 (M39C, A73C, C125A) | APTSSSTKKTQLQLEHLLLDLQMILN GINNYKNPKLTRCLTFKFYMPKKATE LKHLQCLEEELKPLEEVLNLCQSKNF HLRPRDLISNINVIVLELKGSETTFMC EYADETATIVEFLNRWITFAQSIISTLT | 17 | 89 |
| IL-2 Mutan t 16 | IL-2 (R38C, V69C, C125A) | APTSSSTKKTQLQLEHLLLDLQMILN GINNYKNPKLTCMLTFKFYMPKKATE LKHLQCLEEELKPLEECLNLAQSKNF HLRPRDLISNINVIVLELKGSETTFMC EYADETATIVEFLNRWITFAQSIISTLT | 18 | 90 |
| IL-2 Mutan t 17 | IL-2 (R38C, L72C, C125A) | APTSSSTKKTQLQLEHLLLDLQMILN GINNYKNPKLTCMLTFKFYMPKKATE LKHLQCLEEELKPLEEVLNCAQSKNF HLRPRDLISNINVIVLELKGSETTFMC EYADETATIVEFLNRWITFAQSIISTLT | 19 | 91 |
| IL-2 Mutan t 18 | IL-2 (L36C, V69C, C125A) | APTSSSTKKTQLQLEHLLLDLQMILN GINNYKNPKCTRMLTFKFYMPKKAT ELKHLQCLEEELKPLEECLNLAQSKN FHLRPRDLISNINVIVLELKGSETTFM CEYADETATIVEFLNRWITFAQSIIST LT | 20 | 92 |
| IL-2 Mutan t 19 | IL-2 (L36C, L72C, C125A) | APTSSSTKKTQLQLEHLLLDLQMILN GINNYKNPKCTRMLTFKFYMPKKAT ELKHLQCLEEELKPLEEVLNCAQSK NFHLRPRDLISNINVIVLELKGSETTF MCEYADETATIVEFLNRWITFAQSIIS TLT | 21 | 93 |
| IL-2 Mutan t 20 | IL-2 (L36C, A73C, C125A) | APTSSSTKKTQLQLEHLLLDLQMILN GINNYKNPKCTRMLTFKFYMPKKAT ELKHLQCLEEELKPLEEVLNLCQSKN FHLRPRDLISNINVIVLELKGSETTFM CEYADETATIVEFLNRWITFAQSIIST LT | 22 | 94 |
| IL-2 Mutan t 21 | IL-2 (K35C, V69C, C125A) | APTSSSTKKTQLQLEHLLLDLQMILN GINNYKNPCLTRMLTFKFYMPKKAT ELKHLQCLEEELKPLEECLNLAQSKN FHLRPRDLISNINVIVLELKGSETTFM CEYADETATIVEFLNRWITFAQSIIST LT | 23 | 95 |
| IL-2 Mutan t 22 | IL-2 (K43C, A112C, C125A) | APTSSSTKKTQLQLEHLLLDLQMILN GINNYKNPKLTRMLTFCFYMPKKAT ELKHLQCLEEELKPLEEVLNLAQSKN FHLRPRDLISNINVIVLELKGSETTFM CEYADETCTIVEFLNRWITFAQSIIST LT | 24 | 96 |

The expression hosts can be E. *coli* or mammalian cells.

In the second specific embodiment, IL-2 forms a complex with a blocking module through an intermolecular disulfide bond, which completely or partially blocks the binding plane of IL-2 and α receptor, thereby blocking the binding of IL-2 to endogenous α receptor. The disulfide bond between IL-2 and the blocking module was formed by the third cysteine residue introduced into the wild-type IL-2 and the fourth cysteine residue present on or introduced into the blocking module.

In one embodiment, the blocking module was the extracellular segment of α receptor. The amino acid sequence of the extracellular segment of the wild-type α receptor was shown in SEQ ID NO: 25. In the natural state, the binding of α receptor to IL-2 was not stable (having a high dissociation coefficient Kd), and a stable heterodimer cannot be formed. The α receptor must be combined with β and γ receptor subunits to form a high-affinity receptor for IL-2. Therefore, it was impossible to form a stable complex by co-expression of two wild-type molecules. However, through the disulfide bond formed between the third cysteine residue which was introduced into the wild-type IL-2 and the fourth cysteine residue which was introduced into the extracellular segment of the α receptor (CD25-ECD), the IL-2 derivative and the extracellular segment of the α receptor (CD25-ECD) can form a complex to block the binding of IL-2 to the endogenous α receptor.

The amino acid sequence of the extracellular segment of the wild-type α receptor is shown in SEQ ID NO: 25:

ELCDDDPPEIPHATFKAMAYKEGTMLNCECKRGFRRIKSGSLYMLCTGNSSHSSWD
NQCQCTSSATRNTTKQVTPQPEEQKERKTTEMQSPMQPVDQASLPGHCREPPPW
ENEATERIYHFVVGQMVYYQCVQGYRALHRGPAESVCKMTHGKTRWTQPQLICTG.

The nucleotide sequence of the extracellular segment of the wild-type α receptor is shown in SEQ ID NO: 145.

The positions of the third cysteine residue and the fourth cysteine residue to be introduced were mainly determined by the following methods:
1) The positions of the third cysteine residue were the amino acid residues on the binding plane of IL-2 and α receptor or amino acid residues in the vicinity thereof, wherein the amino acid residues on the binding plane of IL-2 and α receptors were the amino acids at positions 37, 38, 41, 42, 43, 44, 45, 61, 62, 65, 68 and 72; and
2) Through design according to bioinformatics and protein engineering, suitable binding plane sites in IL-2 and α receptor extracellular domain (CD25-ECD) were found respectively on the basis of the center-of-mass vector distance of the residues, where the mutation did not affect the structure of the protein.

After the suitable sites are determined, the original amino acid residue on IL-2 was mutated to the third cysteine residue by mutation, and the original amino acid residue on the extracellular domain of the α receptor (CD25-ECD) was mutated to the fourth cysteine residue. After the co-expression of the mutants of IL-2 and extracellular domain of α receptor, disulfide bond can be formed between IL-2 and the extracellular domain of α receptor through transcription and translation, to form a new molecule, IL-2/CD25-ECD heterodimer. The complex cannot bind to the endogenous α receptor in *vivo,* but can bind to the complex of β and γ receptor subunits, so as to achieve the purpose of not activating Treg.

In one embodiment, the original free cysteine at position 125 in IL-2 was mutated to prevent the formation of additional disulfide bond on the extracellular segment of the α receptor with cysteine mutation or IL-2 with cysteine mutation, which affects the formation of the dimer of the α receptor and the IL-2.

In some embodiments, the amino acid sequences of the IL-2 mutants and CD25-ECD mutants obtained through the design are shown in Table 2.

**Table 2. The amino acid sequences of IL-2 mutants and CD25-ECD mutants obtained through the design**

| **Comp lex** | **Mutant and mutation site** | **Amino acid sequence** | **SEQ ID NO:** | **Nucleoti de sequenc e (SEQ ID NO:)** |
|---|---|---|---|---|
| IL-2 Comp lex 1 | IL-2 (C125A, T41C) Pair 1 | APTSSSTKKTQLQLEHLLLDLQMILNGIN NYKNPKLTRMLCFKFYMPKKATELKHLQ CLEEELKPLEEVLNLAQSKNFHLRPRDLI SNINVIVLELKGSETTFMCEYADETATIVE FLNRWITFAQSIISTLT | 26 | 97 |
| | CD25-ECD (N27C) Pair 1 | ELCDDDPPEIPHATFKAMAYKEGTMLCC ECKRGFRRIKSGSLYMLCTGNSSHSSW DNQCQCTSSATRNTTKQVTPQPEEQKE RKTTEMQSPMQPVDQASLPGHCREPPP WENEATERIYHFVVGQMVYYQCVQGYR ALHRGPAESVCKMTHGKTRWTQPQLICT G | 50 | 121 |
| IL-2 Comp lex 2 | IL-2 (C125A, P34C) Pair 2 | APTSSSTKKTQLQLEHLLLDLQMILNGIN NYKNCKLTRMLTFKFYMPKKATELKHLQ CLEEELKPLEEVLNLAQSKNFHLRPRDLI SNINVIVLELKGSETTFMCEYADETATIVE FLNRWITFAQSIISTLT | 27 | 98 |
| | CD25-ECD (D4C) Pair 2 | ELCCDDPPEIPHATFKAMAYKEGTMLNC ECKRGFRRIKSGSLYMLCTGNSSHSSW DNQCQCTSSATRNTTKQVTPQPEEQKE RKTTEMQSPMQPVDQASLPGHCREPPP WENEATERIYHFVVGQMVYYQCVQGYR | 51 | 122 |
| | | ALHRGPAESVCKMTHGKTRWTQPQLICT G | | |
| IL-2 Comp lex 3 | IL-2 (C125A, E68C) Pair 3 | APTSSSTKKTQLQLEHLLLDLQMILNGIN NYKNPKLTRMLTFKFYMPKKATELKHLQ CLEEELKPLECVLNLAQSKNFHLRPRDLI SNINVIVLELKGSETTFMCEYADETATIVE FLNRWITFAQSIISTLT | 28 | 99 |
| | CD25-ECD (L42C) Pair 3 | ELCDDDPPEIPHATFKAMAYKEGTMLNC ECKRGFRRIKSGSCYMLCTGNSSHSSW DNQCQCTSSATRNTTKQVTPQPEEQKE RKTTEMQSPMQPVDQASLPGHCREPPP WENEATERIYHFVVGQMVYYQCVQGYR ALHRGPAESVCKMTHGKTRWTQPQLICT G | 52 | 123 |
| IL-2 Comp lex 4 | IL-2 (C125A, Y45C) Pair 4 | APTSSSTKKTQLQLEHLLLDLQMILNGIN NYKNPKLTRMLTFKFCMPKKATELKHLQ CLEEELKPLEEVLNLAQSKNFHLRPRDLI SNINVIVLELKGSETTFMCEYADETATIVE FLNRWITFAQSIISTLT | 29 | 100 |
| | CD25-ECD (R35C) Pair 4 | ELCDDDPPEIPHATFKAMAYKEGTMLNC ECKRGFCRIKSGSLYMLCTGNSSHSSW DNQCQCTSSATRNTTKQVTPQPEEQKE RKTTEMQSPMQPVDQASLPGHCREPPP WENEATERIYHFVVGQMVYYQCVQGYR ALHRGPAESVCKMTHGKTRWTQPQLICT G | 53 | 124 |
| IL-2 Comp lex 5 | IL-2 (C125A, R38C) Pair 5 | APTSSSTKKTQLQLEHLLLDLQMILNGIN NYKNPKLTCMLTFKFYMPKKATELKHLQ CLEEELKPLEEVLNLAQSKNFHLRPRDLI SNINVIVLELKGSETTFMCEYADETATIVE FLNRWITFAQSIISTLT | 30 | 101 |
| | CD25-ECD (H120C) Pair 5 | ELCDDDPPEIPHATFKAMAYKEGTMLNC ECKRGFRRIKSGSLYMLCTGNSSHSSW DNQCQCTSSATRNTTKQVTPQPEEQKE RKTTEMQSPMQPVDQASLPGHCREPPP WENEATERIYCFVVGQMVYYQCVQGYR ALHRGPAESVCKMTHGKTRWTQPQLICT G | 54 | 125 |
| IL-2 Comp lex 6 | IL-2 (C125A, L72C) Pair 6 | APTSSSTKKTQLQLEHLLLDLQMILNGIN NYKNPKLTCMLTFKFYMPKKATELKHLQ CLEEELKPLEEVLNCAQSKNFHLRPRDLI SNINVIVLELKGSETTFMCEYADETATIVE FLNRWITFAQSIISTLT | 31 | 102 |
| | CD25-ECD (M25C) Pair 6 | ELCDDDPPEIPHATFKAMAYKEGTCLNC ECKRGFRRIKSGSLYMLCTGNSSHSSW DNQCQCTSSATRNTTKQVTPQPEEQKE RKTTEMQSPMQPVDQASLPGHCREPPP WENEATERIYHFVVGQMVYYQCVQGYR ALHRGPAESVCKMTHGKTRWTQPQLICT G | 55 | 126 |
| IL-2 Comp lex 7 | IL-2 (C125A, E61C) Pair 7 | APTSSSTKKTQLQLEHLLLDLQMILNGIN NYKNPKLTRMLTFKFYMPKKATELKHLQ CLECELKPLEEVLNLAQSKNFHLRPRDLI SNINVIVLELKGSETTFMCEYADETATIVE FLNRWITFAQSIISTLT | 32 | 103 |
| | CD25-ECD (S39C) Pair 7 | ELCDDDPPEIPHATFKAMAYKEGTMLNC ECKRGFRRIKCGSLYMLCTGNSSHSSW DNQCQCTSSATRNTTKQVTPQPEEQKE RKTTEMQSPMQPVDQASLPGHCREPPP WENEATERIYHFVVGQMVYYQCVQGYR ALHRGPAESVCKMTHGKTRWTQPQLICT G | 56 | 127 |
| IL-2 Comp lex 8 | IL-2 (C125A, T41C) Pair 8 | APTSSSTKKTQLQLEHLLLDLQMILNGIN NYKNPKLTRMLCFKFYMPKKATELKHLQ CLEEELKPLEEVLNLAQSKNFHLRPRDLI SNINVIVLELKGSETTFMCEYADETATIVE FLNRWITFAQSIISTLT | 33 | 104 |
| | CD25-ECD (I118C) Pair 8 | ELCDDDPPEIPHATFKAMAYKEGTMLNC ECKRGFRRIKSGSLYMLCTGNSSHSSW DNQCQCTSSATRNTTKQVTPQPEEQKE RKTTEMQSPMQPVDQASLPGHCREPPP WENEATERCYHFVVGQMVYYQCVQGY RALHRGPAESVCKMTHGKTRWTQPQLI CTG | 57 | 128 |
| IL-2 Comp lex 9 | IL-2 (C125A, K35C) Pair 9 | APTSSSTKKTQLQLEHLLLDLQMILNGIN NYKNPCLTRMLTFKFYMPKKATELKHLQ CLEEELKPLEEVLNLAQSKNFHLRPRDLI SNINVIVLELKGSETTFMCEYADETATIVE FLNRWITFAQSIISTLT | 34 | 105 |
| | CD25-ECD (D4C) Pair 9 | ELCCDDPPEIPHATFKAMAYKEGTMLNC ECKRGFRRIKSGSLYMLCTGNSSHSSW DNQCQCTSSATRNTTKQVTPQPEEQKE RKTTEMQSPMQPVDQASLPGHCREPPP WENEATERIYHFVVGQMVYYQCVQGYR ALHRGPAESVCKMTHGKTRWTQPQLICT G | 58 | 129 |
| IL-2 Comp lex 10 | IL-2 (C125A, T37C) Pair 10 | APTSSSTKKTQLQLEHLLLDLQMILNGIN NYKNPKLCRMLTFKFYMPKKATELKHLQ CLEEELKPLEEVLNLAQSKNFHLRPRDLI SNINVIVLELKGSETTFMCEYADETATIVE FLNRWITFAQSIISTLT | 35 | 106 |
| | CD25-ECD (D4C) Pair 10 | ELCCDDPPEIPHATFKAMAYKEGTMLNC ECKRGFRRIKSGSLYMLCTGNSSHSSW | 59 | 130 |
| | | DNQCQCTSSATRNTTKQVTPQPEEQKE RKTTEMQSPMQPVDQASLPGHCREPPP WENEATERIYHFVVGQMVYYQCVQGYR ALHRGPAESVCKMTHGKTRWTQPQLICT G | | |
| IL-2 Comp lex 11 | IL-2 (C125A, R38C) Pair 11 | APTSSSTKKTQLQLEHLLLDLQMILNGIN NYKNPKLTCMLTFKFYMPKKATELKHLQ CLEEELKPLEEVLNLAQSKNFHLRPRDLI SNINVIVLELKGSETTFMCEYADETATIVE FLNRWITFAQSIISTLT | 36 | 107 |
| | CD25-ECD (D4C) Pair 11 | ELCCDDPPEIPHATFKAMAYKEGTMLNC ECKRGFRRIKSGSLYMLCTGNSSHSSW DNQCQCTSSATRNTTKQVTPQPEEQKE RKTTEMQSPMQPVDQASLPGHCREPPP WENEATERIYHFVVGQMVYYQCVQGYR ALHRGPAESVCKMTHGKTRWTQPQLICT G | 60 | 131 |
| IL-2 Comp lex 12 | IL-2 (C125A, R38C) Pair 12 | APTSSSTKKTQLQLEHLLLDLQMILNGIN NYKNPKLTCMLTFKFYMPKKATELKHLQ CLEEELKPLEEVLNLAQSKNFHLRPRDLI SNINVIVLELKGSETTFMCEYADETATIVE FLNRWITFAQSIISTLT | 37 | 108 |
| | CD25-ECD (D5C) Pair 12 | ELCDCDPPEIPHATFKAMAYKEGTMLNC ECKRGFRRIKSGSLYMLCTGNSSHSSW DNQCQCTSSATRNTTKQVTPQPEEQKE RKTTEMQSPMQPVDQASLPGHCREPPP WENEATERIYHFVVGQMVYYQCVQGYR ALHRGPAESVCKMTHGKTRWTQPQLICT G | 61 | 132 |
| IL-2 Complex 13 | IL-2 (C125A, T41C) Pair 13 | APTSSSTKKTQLQLEHLLLDLQMILNGIN NYKNPKLTRMLCFKFYMPKKATELKHLQ | 38 | 109 |
| | | CLEEELKPLEEVLNLAQSKNFHLRPRDLI SNINVIVLELKGSETTFMCEYADETATIVE FLNRWITFAQSIISTLT | | |
| | CD25-ECD (L42C) Pair 13 | ELCDDDPPEIPHATFKAMAYKEGTMLNC ECKRGFRRIKSGSCYMLCTGNSSHSSW DNQCQCTSSATRNTTKQVTPQPEEQKE RKTTEMQSPMQPVDQASLPGHCREPPP WENEATERIYHFVVGQMVYYQCVQGYR ALHRGPAESVCKMTHGKTRWTQPQLICT G | 62 | 133 |
| IL-2 Comp lex 14 | IL-2 (C125A, T41C) Pair 14 | APTSSSTKKTQLQLEHLLLDLQMILNGIN NYKNPKLTRMLCFKFYMPKKATELKHLQ CLEEELKPLEEVLNLAQSKNFHLRPRDLI SNINVIVLELKGSETTFMCEYADETATIVE FLNRWITFAQSIISTLT | 39 | 110 |
| | CD25-ECD (Y119C) Pair 14 | ELCDDDPPEIPHATFKAMAYKEGTMLNC ECKRGFRRIKSGSLYMLCTGNSSHSSW DNQCQCTSSATRNTTKQVTPQPEEQKE RKTTEMQSPMQPVDQASLPGHCREPPP WENEATERICHFVVGQMVYYQCVQGYR ALHRGPAESVCKMTHGKTRWTQPQLICT G | 63 | 134 |
| IL-2 Comp lex 15 | IL-2 (C125A, K43C) Pair 15 | APTSSSTKKTQLQLEHLLLDLQMILNGIN NYKNPKLTRMLTFCFYMPKKATELKHLQ CLEEELKPLEEVLNLAQSKNFHLRPRDLI SNINVIVLELKGSETTFMCEYADETATIVE FLNRWITFAQSIISTLT | 40 | 111 |
| | CD25-ECD (R35C) Pair 15 | ELCDDDPPEIPHATFKAMAYKEGTMLNC ECKRGFCRIKSGSLYMLCTGNSSHSSW DNQCQCTSSATRNTTKQVTPQPEEQKE RKTTEMQSPMQPVDQASLPGHCREPPP | 64 | 135 |
| | | WENEATERIYHFVVGQMVYYQCVQGYR ALHRGPAESVCKMTHGKTRWTQPQLICT G | | |
| IL-2 Comp lex 16 | IL-2 (C125A, K43C) Pair 16 | APTSSSTKKTQLQLEHLLLDLQMILNGIN NYKNPKLTRMLTFCFYMPKKATELKHLQ CLEEELKPLEEVLNLAQSKNFHLRPRDLI SNINVIVLELKGSETTFMCEYADETATIVE FLNRWITFAQSIISTLT | 41 | 112 |
| | CD25-ECD (R36C) Pair 16 | ELCDDDPPEIPHATFKAMAYKEGTMLNC ECKRGFRCIKSGSLYMLCTGNSSHSSW DNQCQCTSSATRNTTKQVTPQPEEQKE RKTTEMQSPMQPVDQASLPGHCREPPP WENEATERIYHFVVGQMVYYQCVQGYR ALHRGPAESVCKMTHGKTRWTQPQLICT G | 65 | 136 |
| IL-2 Comp lex 17 | IL-2 (C125A, F44C) Pair 17 | APTSSSTKKTQLQLEHLLLDLQMILNGIN NYKNPKLTRMLTFKCYMPKKATELKHLQ CLEEELKPLEEVLNLAQSKNFHLRPRDLI SNINVIVLELKGSETTFMCEYADETATIVE FLNRWITFAQSIISTLT | 42 | 113 |
| | CD25-ECD (L42C) Pair 17 | ELCDDDPPEIPHATFKAMAYKEGTMLNC ECKRGFRRIKSGSCYMLCTGNSSHSSW DNQCQCTSSATRNTTKQVTPQPEEQKE RKTTEMQSPMQPVDQASLPGHCREPPP WENEATERIYHFVVGQMVYYQCVQGYR ALHRGPAESVCKMTHGKTRWTQPQLICT G | 66 | 137 |
| IL-2 Comp lex 18 | IL-2 (C125A, K43C) Pair 18 | APTSSSTKKTQLQLEHLLLDLQMILNGIN NYKNPKLTRMLTFCFYMPKKATELKHLQ CLEEELKPLEEVLNLAQSKNFHLRPRDLI SNINVIVLELKGSETTFMCEYADETATIVE | 43 | 114 |
| | | FLNRWITFAQSIISTLT | | |
| | CD25-ECD (L42C) Pair 18 | ELCDDDPPEIPHATFKAMAYKEGTMLNC ECKRGFRRIKSGSCYMLCTGNSSHSSW DNQCQCTSSATRNTTKQVTPQPEEQKE RKTTEMQSPMQPVDQASLPGHCREPPP WENEATERIYHFVVGQMVYYQCVQGYR ALHRGPAESVCKMTHGKTRWTQPQLICT G | 67 | 138 |
| IL-2 Comp lex 19 | IL-2 (C125A, E61C) Pair 19 | APTSSSTKKTQLQLEHLLLDLQMILNGIN NYKNPKLTRMLTFKFYMPKKATELKHLQ CLECELKPLEEVLNLAQSKNFHLRPRDLI SNINVIVLELKGSETTFMCEYADETATIVE FLNRWITFAQSIISTLT | 44 | 115 |
| | CD25-ECD (K38C) Pair 19 | ELCDDDPPEIPHATFKAMAYKEGTMLNC ECKRGFRRICSGSLYMLCTGNSSHSSW DNQCQCTSSATRNTTKQVTPQPEEQKE RKTTEMQSPMQPVDQASLPGHCREPPP WENEATERIYHFVVGQMVYYQCVQGYR ALHRGPAESVCKMTHGKTRWTQPQLICT G | 68 | 139 |
| IL-2 Comp lex 20 | IL-2 (C125A, E62C) Pair 20 | APTSSSTKKTQLQLEHLLLDLQMILNGIN NYKNPKLTRMLTFKFYMPKKATELKHLQ CLEECLKPLEEVLNLAQSKNFHLRPRDLI SNINVIVLELKGSETTFMCEYADETATIVE FLNRWITFAQSIISTLT | 45 | 116 |
| | CD25-ECD (K38C) Pair 20 | ELCDDDPPEIPHATFKAMAYKEGTMLNC ECKRGFRRICSGSLYMLCTGNSSHSSW DNQCQCTSSATRNTTKQVTPQPEEQKE RKTTEMQSPMQPVDQASLPGHCREPPP WENEATERIYHFVVGQMVYYQCVQGYR ALHRGPAESVCKMTHGKTRWTQPQLICT | 69 | 140 |
| | | G | | |
| IL-2 Comp lex 21 | IL-2 (C125A, K64C) Pair 21 | APTSSSTKKTQLQLEHLLLDLQMILNGIN NYKNPKLTRMLTFKFYMPKKATELKHLQ CLEEELCPLEEVLNLAQSKNFHLRPRDLI SNINVIVLELKGSETTFMCEYADETATIVE FLNRWITFAQSIISTLT | 46 | 117 |
| | CD25-ECD (S39C) Pair 21 | ELCDDDPPEIPHATFKAMAYKEGTMLNC ECKRGFRRIKCGSLYMLCTGNSSHSSW DNQCQCTSSATRNTTKQVTPQPEEQKE RKTTEMQSPMQPVDQASLPGHCREPPP WENEATERIYHFVVGQMVYYQCVQGYR ALHRGPAESVCKMTHGKTRWTQPQLICT G | 70 | 141 |
| IL-2 Comp lex 22 | IL-2 (C125A, K64C) Pair 22 | APTSSSTKKTQLQLEHLLLDLQMILNGIN NYKNPKLTRMLTFKFYMPKKATELKHLQ CLEEELCPLEEVLNLAQSKNFHLRPRDLI SNINVIVLELKGSETTFMCEYADETATIVE FLNRWITFAQSIISTLT | 47 | 118 |
| | CD25-ECD (G40C) Pair 22 | ELCDDDPPEIPHATFKAMAYKEGTMLNC ECKRGFRRIKSCSLYMLCTGNSSHSSW DNQCQCTSSATRNTTKQVTPQPEEQKE RKTTEMQSPMQPVDQASLPGHCREPPP WENEATERIYHFVVGQMVYYQCVQGYR ALHRGPAESVCKMTHGKTRWTQPQLICT G | 71 | 142 |
| IL-2 Comp lex 23 | IL-2 (C125A, K64C) Pair 23 | APTSSSTKKTQLQLEHLLLDLQMILNGIN NYKNPKLTRMLTFKFYMPKKATELKHLQ CLEEELCPLEEVLNLAQSKNFHLRPRDLI SNINVIVLELKGSETTFMCEYADETATIVE FLNRWITFAQSIISTLT | 48 | 119 |
| | CD25-ECD (S41C) Pair 23 | ELCDDDPPEIPHATFKAMAYKEGTMLNC ECKRGFRRIKSGCLYMLCTGNSSHSSW DNQCQCTSSATRNTTKQVTPQPEEQKE RKTTEMQSPMQPVDQASLPGHCREPPP WENEATERIYHFVVGQMVYYQCVQGYR ALHRGPAESVCKMTHGKTRWTQPQLICT G | 72 | 143 |
| IL-2 Comp lex 24 | IL-2 (C125A, P65C) Pair 24 | APTSSSTKKTQLQLEHLLLDLQMILNGIN NYKNPKLTRMLTFKFYMPKKATELKHLQ CLEEELKCLEEVLNLAQSKNFHLRPRDLI SNINVIVLELKGSETTFMCEYADETATIVE FLNRWITFAQSIISTLT | 49 | 120 |
| | CD25-ECD (K38C) Pair 24 | ELCDDDPPEIPHATFKAMAYKEGTMLNC ECKRGFRRICSGSLYMLCTGNSSHSSW DNQCQCTSSATRNTTKQVTPQPEEQKE RKTTEMQSPMQPVDQASLPGHCREPPP WENEATERIYHFVVGQMVYYQCVQGYR ALHRGPAESVCKMTHGKTRWTQPQLICT G | 73 | 144 |

The expression hosts were mammalian cells (HEK293 or CHO).

### Example 1: Preparation of IL-2 (C125A), IL-2 mutants and IL-2 complexes

In this example, IL-2 wt (C125A), IL-2 mutants 1-4 and IL-2 complexes 1-4 were selected for expression, respectively. The HPC4 tag attached to the C-terminus of the molecule was used for purification and preparation.

### 1.1 Construction of expression plasmid

The genes of IL-2 wt C125A (SEQ ID NO: 74), IL-2 mutants 1-4, and IL-2 complexes 1-4 (IL-2 Pair 1-4 and CD25-ECD Pair 1-4 for IL-2 complexes) were synthesized by Suzhou Jinweizhi Biotechnology Co., Ltd. Then the overlapping PCR was performed in accordance with the procedures as described in "Molecular Cloning" to obtain the target fragments. Subsequently, the fragments and pTT5 universal vectors were recombined, ligated, transformed (DH10B), sequenced and preserved to obtain the desired plasmids of the IL-2 wt (C125A), IL-2 mutants 1-4 and IL-2 complexes 1-4 (plasmids of IL-2 Pair 1-4 and CD25-ECD Pair 1-4 for the IL-2 complexes). Or PCR, Dpnl digestion, DH10B transformation, sequencing, and bacteria preservation were performed in accordance with the procedures as described in "Agilent Quik Change Lightning Site - Directed Mutagenesis Kit" to obtain the desired plasmids of IL-2 wt (C125A), IL-2 mutants 1-4 and IL-2 complexes 1-4.

### 1.2 Extraction of plasmids and preparation of HEK293 cells

### 1.2.1 Extraction of plasmids

The preparation of the plasmids of IL-2 wt (C125A), IL-2 mutants 1-4 and IL-2 complexes 1-4 was performed in accordance with the procedures as described in "Qiagen Mini-prep Kit" and "Qiagen Endofree Maxi-prep Kit".

### 1.2.2 Preparation of HEK293 cells

Freshly passaged HEK293 cells (National Research Council, Canada) with a density of 1 to 1.2×10⁶ cells/mL were used for transient expression.

### 1.3 Transient expression of HEK293

### 1.3.1 Preparation of reagents

A) G418 solution: 250 mg of Geneticin^{™} was weighed, dissolved in 4.5 mL ultrapure water, added with ultrapure water to give a volume of 5 mL, filtered with a 0.22 µm filter membrane, and stored at -20 °C;
B) PEI solution: 50 mg of PEI was weighed, dissolved in 4.5 mL ultrapure water, adjusted with 1 M NaOH to pH 7.0, added with ultrapure water to give a volume of 50 mL, filtered with a 0.22 µm filter membrane, and store at -20 °C;
C) Medium: 10 mL of Pluronicd^{™} F-68 and 500 µL of G418 were added to 1 L of FreeStyle^{™} 293 Expression Medium;
D) The plasmid was prepared in advance in a 2 mL de-endotoxin centrifuge tube; and
E) A freshly passaged cell suspension of 1 to 1.2×10⁶ cells/mL was prepared according to the volume required for transfection.

### 1.3.2 Preparation of transfection reagent - plasmid complex

Liquid A: Plasmid 1 µg/mL + Opti-MEM^{™} 33.3 µL/mL
Liquid B: PEI 2 pg/mL + Opti-MEM^{™} 33.3 pL/mL
Liquid B was introduced into Liquid A and incubated for 10 minutes. Then the cell suspension was added.

During transfection, plasmids of IL-2 wt (C125A) and IL-2 mutants 1-4 were transfected separately; and the plasmids of IL-2 complexes 1-4 were transfected after mixing the two plasmids.

### 1.3.3 Replacement of liquids

After incubating at 115rpm, 36.8 °C and 5% CO₂ for 4 h, the culture was centrifuged at 800g for 5 minutes and replaced with FreeStyle^{™} 293 Expression Medium free of F68 and G418.

### 1.3.4 Expression of the culture and harvest

After incubating at 115rpm, 36.8 °C and 5% CO₂ for 5 days, the culture was centrifuged at 8500 rpm for 15 minutes and the cell supernatant was collected.

### 1.4 Preparation of purified product

All the derivatives related to IL-2 had an HPC4 tag at the C-terminus. Therefore, they could be affinity purified with fillers coupled with HPC4 antibody, and then further purified by gel filtration chromatography (Superdex 200) to obtain proteins with higher purity. SDS-PAGE analysis was performed according to the method as described in *Molecular Cloning.*

The results were shown in Fig. 1, indicating that after the plasmids were transfected into the cells, the corresponding proteins and complexes of IL-2 wt (C125A), IL-2 mutants 1-4 and IL-2 complexes 1-4 were produced. The construction of plasmids, and the expression and purification of proteins were successful.

### Example 2: Determination of the affinity of IL-2 wt (C125A), IL-2 mutants 1-4 and IL-2 complexes 1-4 to IL2Rβγ and IL2Ra by biolayer interferomeory (BLI)

### 1. Experimental Materials

The proteins used in the experiment were produced by Beijing Zhidao Biotechnology Co., Ltd. IL2Rα-his (available from Beijing Zhidao Biotechnology Co., Ltd.), IL2Rβγ-Fc (available from Beijing Zhidao Biotechnology Co., Ltd.) and IL-2 mutants were obtained by transient expression with HEK293 and affinity purification. The buffer formulation was as follows: 10 mM HEPES, 150 mM sodium chloride, 3 mM EDTA, 0.1 % BSA and 0.05% Tween 20. The ProA sensor was purchased from Pall Fortebio (Cat. No. #18-5010). The HISIK sensor was purchased from Pall Fortebio (Cat. No. #18-5120). The BLI equipment was Octet RED96 from Pall Fortebio. Data acquisition and analysis were carried out using software Data acquisition 11.0 and Data Analysis 11.0, respectively.

### 2. Experimental methods

### 1) Preparation of IL2Rβγ-Fc

IL2Rβγ-Fc was diluted with the buffer solution to a concentration of 10 µg/mL, and then added to column 2 of the 96-well assay plate. The program was set to Loading, 600s.

### 2) Preparation of IL2Rα-his

IL2Rα-his was diluted with the buffer solution to a concentration of 10 µg/mL, and then added to column 3 of the 96-well assay plate. The program was set to Loading, 600s.

### 3) Preparation of samples

The IL-2 derivative was diluted with the buffer solution to 100 nM, and then serially diluted downward by 6 gradients (7 gradients in total) at a ratio of 1:1 to a concentration of 1.625 nM and a concentration of 0 nM. They were added to columns 5-9 of the 96-well assay plate, respectively. The program was set to Association, 200s. The buffers were added to columns 1, 4, 10 and 11 of the 96-well assay plate, and the glycine with pH 1.7 was added to column 12. The loading volume of the above samples or solutions was 200 µL.

### 4) Detection of affinity with IL2Rβγ-Fc

8 ProA sensors were respectively placed in A-H of column 1 of the sensor holder. In the software Data acquisition 11.0, the detection conditions were set as follows. 1 Prewetting: Baseline, 60s, Position: Column 1. 2 Cycle detection: column 2: loading, 600s; column 4: Baseline 1, 60s; columns 5-9: Association, 200s; column 10: Dissociation, 600s; column 11: neutralization; and column 12: regeneration.

### 5) Detection of binding to IL2Rα

8 HISIK sensors were respectively placed in A-H of column 2 of the sensor holder. In the software Data acquisition 11.0, the detection conditions were set as follows. 1 prewet: Baseline, 60s, position: column 1. 2 Cycle detection: column 3: loading, 600s; column 4: Baseline 1, 60s; columns 5-9: Association, 200s; column 10: Dissociation, 600s; column 11: neutralization; and column 12: regeneration.

### 6) Data analysis

The software Data Analysis 11.0 was used to analyze the data. KD value was calculated by Fitting curve, and concentration 0 was used as the control to subtract background.

### 3. Results

In Fig. 2, as can be seen from the binding curves of IL-2 derivatives and IL2Ra receptors, all the IL-2 derivatives hardly bond to IL2Ra receptors, except that the binding of IL-2 mutant 1 to IL2Ra receptor was greatly weakened. In Fig. 3, as can be seen from the binding curves of IL-2 derivatives and IL2Rβγ receptors, there was no significant change compared with IL-2 wt C125A (Fig. 3).

### Example 3: Proliferation test of T cells

The proliferation test of CTLL-2 (T cells) was a commonly used cell-level assay to determine the activity of interleukins to stimulate immune cells. Therefore, the proliferation test of CTLL-2 cells was used to examine the biological activity of the IL-2 derivatives.
1) Preparation of CTLL-2 cells: The cells were resuspended in culture medium containing FBS and Rat-T-Stim.
2) Loading of samples: The cells were seeded in a 96-well culture plate with 0.1 mL per well.
   At the same time, the samples of proteins of IL-2 derivatives 11, 18, 21 and 28 (i.e., proteins prepared in Example 1) to be tested were diluted in multiples. 0.1 mL was added to each well, and 3 replicate wells were set for each dilution concentration. In addition, the culture media control well (100 µL cells + 100 µL culture media) was also set. They were incubated at 37 °C and 5% CO₂ for 72 hours.
3) MTS addition: 20 µL of CellTiter96^{®} AQueous One Solution Reagent was added to each well, and incubated at 37 °C and 5% CO₂ for 2 to 4 hours.
4) Detection: The absorbance (A) was detected with a microplate reader at a wavelength of 490 nm and the EC₅₀ value was calculated.
5) Results: Representative IL-2 complex 2 and IL-2 mutant 4 were selected, all of which had the proliferation activity of CTLL-2 (T cell). It indicated that the signal transduction function of the complexes of β and γ receptor subunits was not seriously affected (Fig. 4).

## Claims

1. An IL-2 derivative, **characterized in that** the IL-2 derivative is introduced with at least one cysteine residue based on wild-type IL-2, and the binding plane of the IL-2 derivative and α receptor subunit is partially or completely blocked, while the affinity of the IL-2 derivative to the complex of β and γ receptor subunits is basically retained.

2. The IL-2 derivative according to claim 1, **characterized in that** the at least one cysteine residue introduced based on wild-type IL-2 is capable of:
a) forming an intramolecular disulfide bond of the IL-2 derivative; or
b) enabling the IL-2 derivative to bind to a blocking module through an intermolecular disulfide bond.

3. The IL-2 derivative according to claim 2, **characterized in that** the at least one cysteine residue is introduced by means of point mutation.

4. The IL-2 derivative according to claim 3, **characterized in that** a first cysteine residue and a second cysteine residue are introduced by means of point mutation based on wild-type IL-2; and one or both of the first cysteine residue and the second cysteine residue are amino acids related to the binding plane of the wild-type IL-2 and α receptor subunit, or amino acids in the vicinity thereof.

5. The IL-2 derivative according to claim 4, **characterized in that** the first cysteine residue is an amino acid at position 37, position 38, position 41, position 42, position 43, position 44, position 45, position 61 or position 62 of the wild-type IL-2, or an amino acid in the vicinity thereof; and the second cysteine residue is an amino acid at position 61, position 62, position 65, position 68 or position 72 of the wild-type IL-2, or an amino acid in the vicinity thereof.

6. The IL-2 derivative according to claim 4, **characterized in that** the first cysteine residue is an amino acid point mutant selected from the group consisting of: K35C, L36C, R38C, M39C, L40C, T41C, F42C, K43C, F44C and E61C.

7. The IL-2 derivative according to claim 4, **characterized in that** the second cysteine residue is an amino acid point mutant selected from the group consisting of: V69C, E62C, P65C, T111C, Y107C, A112C, T113C, 1114C, L72C andA73C.

8. The IL-2 derivative according to claim 4, **characterized in that** a combination of the first cysteine residue and the second cysteine residue that form the intramolecular disulfide bond is a combination of amino acid point mutants selected from the group consisting of: M39C and V69C; F44C and E62C; F44C and P65C; F42C and V69C; E61C and Y107C; F42C and P65C; F42C and T111C; F42C and A112C; F42C and T113C; T41C and A112C; L40C and A112C; T113C and T113C; L40C and I114C; M39C and L72C; M39C and A73C; R38C and V69C; R38C and L72C; L36C and V69C; L36C and L72C; L36C and A73C; K35C and V69C; and K43C and A112C.

9. The IL-2 derivative according to claim 4, **characterized in that** a center-of-mass vector distance between the first cysteine residue and the second cysteine residue is less than 6Å.

10. The IL-2 derivative according to claim 3, **characterized in that** a third cysteine residue is introduced by means of point mutation based on wild-type IL-2; and the third cysteine residue is an amino acid related to the binding plane of wild-type IL-2 and α receptor subunit, or an amino acid in the vicinity thereof.

11. The IL-2 derivative according to claim 10, **characterized in that** the third cysteine residue is an amino acid at position 37, position 38, position 41, position 42, position 43, position 44, position 45, position 61, position 62, position 65, position 68 or position 72 of the wild-type IL-2, or an amino acid in the vicinity thereof.

12. The IL-2 derivative according to claim 10, **characterized in that** the third cysteine residue is an amino acid point mutant selected from the group consisting of: P34C, K35C, T37C, R38C, T41C, K43C, F44C, Y45C, E61C, E62C, K64C, P65C, E68C and L72C.

13. The IL-2 derivative according to claim 10, **characterized in that** the blocking module has or is introduced with a fourth cysteine residue; and the third cysteine residue on the IL-2 derivative and the fourth cysteine residue on the blocking module are capable of forming an intermolecular disulfide bond.

14. The IL-2 derivative according to claim 13, **characterized in that** a center-of-mass vector distance between the third cysteine residue and the fourth cysteine residue is less than 6Å.

15. The IL-2 derivative according to claim 13, **characterized in that** the blocking module is an extracellular segment of the α receptor subunit.

16. The IL-2 derivative according to claim 3, **characterized in that** the cysteine residue at position 125 of the wild-type IL-2 is converted into another amino acid residue by means of point mutation.

17. The IL-2 derivative according to claim 16, **characterized in that** a point mutant at position 125 of the wild-type IL-2 is C125A.

18. The IL-2 derivative according to claim 3, **characterized in that** the amino acid sequence of the IL-2 derivative is shown in SEQ ID NO: 3 to SEQ ID NO: 24, or in SEQ ID NO: 26 to SEQ ID NO: 40.

19. A complex, comprising:
1) an IL-2 derivative, which is introduced with a third cysteine residue based on wild-type IL-2; and
2) a blocking module, which has or is introduced with a fourth cysteine residue;
wherein the third cysteine residue on the IL-2 derivative and the fourth cysteine residue on the blocking module are capable of forming an intermolecular disulfide bond, thereby forming a complex of the IL-2 derivative and the blocking module; and wherein the binding plane of the IL-2 derivative and α receptor subunit is partially or completely blocked, while the affinity of the IL-2 derivative to the complex of β and γ receptor subunits is basically retained.

20. The complex according to claim 19, **characterized in that** the third cysteine residue is an amino acid related to the binding plane of the wild-type IL-2 and the α receptor subunit, or an amino acid in the vicinity thereof.

21. The complex according to claim 20, **characterized in that** a center-of-mass vector distance between the third cysteine residue and the fourth cysteine residue is less than 6Å.

22. The complex according to claim 19, **characterized in that** the third cysteine residue is an amino acid point mutant selected from the group consisting of: P34C, K35C, T37C, R38C, T41C, K43C, F44C, Y45C, E61C, E62C, K64C, P65C, E68C and L72C.

23. The complex according to claim 19, **characterized in that** the blocking module is an extracellular segment of the α receptor subunit, the amino acid sequence of which is shown in SEQ ID NO: 25.

24. The complex according to claim 23, **characterized in that** the fourth cysteine residue on the extracellular segment of the α receptor subunit is an amino acid point mutant selected from the group consisting of: D4C, D5C, M25C, N27C, R35C, R36C, K38C, S39C, G40C, S41C, L42C, I118C, Y119C and H120C.

25. The complex according to claim 23, **characterized in that** a combination of the third cysteine residue and the fourth cysteine residue that form the intramolecular disulfide bond is a combination of amino acid point mutants selected from the group consisting of: T41C and N27C; P34C and D4C; E68C and L42C; Y45C and R35C; R38C and H120C; L72C and M25C; E61C and S39C; T41C and I118C; K35C and D4C; T37C and D4C; R38C and D4C; R38C and D5C; T41C and L42C; T41C and Y119C; K43C and R35C; K43C and R36C; F44C and L42C; K43C and L42C; E61C and K38C; E62C and K38C; K64C and S39C; K64C and G40C; K64C and S41C; and P65C and K38C.

26. The complex according to claim 19, **characterized in that** a point mutant at position 125 of the wild-type IL-2 is C125A.

27. The complex according to claim 23, **characterized in that** a combination of the amino acid sequence of the IL-2 derivative and the sequence of the extracellular segment of the α receptor subunit is a combination selected from the group consisting of: SEQ ID NO: 26 and SEQ ID NO: 50; SEQ ID NO: 27 and SEQ ID NO: 51; SEQ ID NO: 28 and SEQ ID NO: 52; SEQ ID NO: 29 and SEQ ID NO: 53; SEQ ID NO: 30 and SEQ ID NO: 54; SEQ ID NO: 31 and SEQ ID NO: 55; SEQ ID NO: 32 and SEQ ID NO: 56; SEQ ID NO: 33 and SEQ ID NO: 57; SEQ ID NO: 34 and SEQ ID NO: 58; SEQ ID NO: 35 and SEQ ID NO: 59; SEQ ID NO: 36 and SEQ ID NO: 60; SEQ ID NO: 37 and SEQ ID NO: 61; SEQ ID NO: 38 and SEQ ID NO: 62; SEQ ID NO: 39 and SEQ ID NO: 63; SEQ ID NO: 40 and SEQ ID NO: 64; SEQ ID NO: 41 and SEQ ID NO: 65; SEQ ID NO: 42 and SEQ ID NO: 66; SEQ ID NO: 43 and SEQ ID NO: 67; SEQ ID NO: 44 and SEQ ID NO: 68; SEQ ID NO: 45 and SEQ ID NO: 69; SEQ ID NO: 46 and SEQ ID NO: 70; SEQ ID NO: 47 and SEQ ID NO: 71; SEQ ID NO: 48 and SEQ ID NO: 72; and SEQ ID NO: 49 and SEQ ID NO: 73.

28. An isolated polynucleotide, which encodes the IL-2 derivative according to any one of claims 1-18 or the complex according to any one of claims 19-27.

29. An expression vector, comprising the isolated polynucleotide according to claim 28.

30. A host cell, comprising the isolated polynucleotide according to claim 28.

31. A composition, comprising the IL-2 derivative according to any one of claims 1 to 18 or the complex according to any one of claims 19 to 27, and a pharmaceutically acceptable carrier.

32. Use of the IL-2 derivative according to any one of claims 1 to 18 or the complex according to any one of claims 19 to 27 for preparing drugs or preparations for treating diseases.

33. Use of the IL-2 derivative according to any one of claims 1 to 18 or the complex according to any one of claims 19 to 27 for preparing a composition for stimulating the immune system of an individual.

34. A method for producing an IL-2 derivative, which includes culturing the host cell according to claim 30 under conditions suitable for expressing the IL-2 derivative.

35. A method for producing a complex, which includes culturing the host cell according to claim 30 under conditions suitable for expressing the complex.
